# EUROPEAN PATENT APPLICATION

(11) **EP 4 834 200 A1**
(43) Date of publication of application: **04.11.2026**
(21) Application number: 24912231.8
(22) Date of filing: 03.12.2024
(51) Int. Cl.: A61B 3/028, A61B 3/09, A61B 3/103, A61B 3/113, A61B 5/107, G02C 13/00

(54) **TERMINAL DEVICE, ABERRATION SENSITIVITY INFORMATION GENERATION METHOD, AND PROGRAM**

(30) Priority: 25.12.2023 JP 2023217569
(71) Applicant: Nikon-Essilor Co., Ltd., Tokyo 130-0026 (JP)
(72) Inventor: KISHIMOTO Takeshi, Tokyo 130-0026 (JP)
(74) Representative: Hoffmann Eitle
(86) International application number: PCT/JP2024/042725
(87) International publication number: WO 2025/142341

(57) **Abstract**

A terminal device includes a first image output unit configured to output image information of an image for a left eye, in which a first aberration distribution as an aberration distribution for a left eye is settable, to a left eye display unit configured to present an image to a left eye of a subject, a second image output unit configured to output image information of an image for a right eye, in which a second aberration distribution as an aberration distribution for a right eye is settable, to a right eye display unit configured to present an image to a right eye of the subject, a line-of-sight information acquisition unit configured to acquire line-of-sight information obtained by detecting movement of a line of sight of the subject presented with at least one of the image for the left eye and the image for the right eye, and an aberration sensitivity information generation unit configured to generate aberration sensitivity information, which is information regarding sensitivity of the subject to aberration, on the basis of the acquired line-of-sight information and the aberration distribution.

## Description

### TECHNICAL FIELD

The present invention relates to a terminal device, an aberration sensitivity information generation method, and a program.

Priority is claimed on Japanese Patent Application No. 2023-217569, filed December 25, 2023, the content of which is incorporated herein by reference.

### BACKGROUND ART

In the related art, a technique for enabling evaluation of viewing performance of a spectacle lens for each wearer of spectacles is disclosed (for example, see Patent Document 1).

### Citation List

### Patent Document

Patent Document 1: Japanese Unexamined Patent Application, First Publication No. 2003-177076

### SUMMARY OF INVENTION

An embodiment of the present invention is a terminal device including a first image output unit configured to output image information of an image for a left eye, in which a first aberration distribution as an aberration distribution for a left eye is settable, to a left eye display unit configured to present an image to a left eye of a subject, a second image output unit configured to output image information of an image for a right eye, in which a second aberration distribution as an aberration distribution for a right eye is settable, to a right eye display unit configured to present an image to a right eye of the subject, a line-of-sight information acquisition unit configured to acquire line-of-sight information obtained by detecting movement of a line of sight of the subject presented with at least one of the image for the left eye and the image for the right eye, and an aberration sensitivity information generation unit configured to generate aberration sensitivity information, which is information regarding sensitivity of the subject to aberration, on the basis of the acquired line-of-sight information and the aberration distribution.

An embodiment of the present invention is an aberration sensitivity information generation method in which a computer executes outputting image information of an image for a left eye, in which a first aberration distribution as an aberration distribution for a left eye is settable, to a left eye display unit configured to present an image to a left eye of a subject, outputting image information of an image for a right eye, in which a second aberration distribution as an aberration distribution for a right eye is settable, to a right eye display unit configured to present an image to a right eye of the subject, acquiring line-of-sight information obtained by detecting movement of a line of sight of the subject presented with at least one of the image for the left eye and the image for the right eye, and generating aberration sensitivity information, which is information regarding sensitivity of the subject to aberration, on the basis of the acquired line-of-sight information and the aberration distribution.

An embodiment of the present invention is a program causing a computer to execute outputting image information of an image for a left eye, in which a first aberration distribution as an aberration distribution for a left eye is settable, to a left eye display unit configured to present an image to a left eye of a subject, outputting image information of an image for a right eye, in which a second aberration distribution as an aberration distribution for a right eye is settable, to a right eye display unit configured to present an image to a right eye of the subject, acquiring line-of-sight information obtained by detecting movement of a line of sight of the subject presented with at least one of the image for the left eye and the image for the right eye, and generating aberration sensitivity information, which is information regarding sensitivity of the subject to aberration, on the basis of the acquired line-of-sight information and the aberration distribution.

### BRIEF DESCRIPTION OF DRAWINGS

[FIG. 1] A perspective view showing an external appearance of a display device of a present embodiment.
[FIG. 2] A diagram schematically showing a cross section of a main body of the display device.
[FIG. 3] A diagram illustrating an Euler angle.
[FIG. 4] A diagram showing an example of a functional configuration of an aberration sensitivity measurement system of the present embodiment.
[FIG. 5] A diagram showing an example of a flow of an operation of sensitivity evaluation by the aberration sensitivity measurement system of the present embodiment.
[FIG. 6] A diagram showing a first example of a measurement scene in a virtual space of the present embodiment.
[FIG. 7] A diagram showing an example of an image to which blur aberration presented by the aberration sensitivity measurement system of the present embodiment is applied.
[FIG. 8] A diagram showing a second example of a measurement scene in the virtual space of the present embodiment.
[FIG. 9] A diagram showing a third example of a measurement scene in the virtual space of the present embodiment.
[FIG. 10] A diagram showing a fourth example of a measurement scene in the virtual space of the present embodiment.
[FIG. 11] A diagram showing a fifth example of a measurement scene in the virtual space of the present embodiment.
[FIG. 12] A diagram showing an example of a line-of-sight guide in the virtual space of the present embodiment.
[FIG. 13] A diagram showing a sixth example of a measurement scene in the virtual space of the present embodiment.
[FIG. 14] A diagram showing an example of a clipping plane of a viewing frustum.
[FIG. 15] A diagram showing an example of a coordinate system of a near clipping plane.
[FIG. 16] A diagram showing an example of a texture coordinate system.
[FIG. 17] A diagram showing an example of an image processing coordinate system.
[FIG. 18] A diagram showing an example of conversion from an angle into normalization coordinates of a clipping plane.
[FIG. 19] A diagram showing an example of conversion from normalization coordinates of a clipping plane to an angle.
[FIG. 20] A diagram showing an example of a virtual space in obtaining a center of an aberration distribution of the present embodiment.
[FIG. 21] An example of change with time of an intersection position between a direction of a line of sight of a subject and the near clipping plane of the viewing frustum.
[FIG. 22] An example of a distribution of line-of-sight center coordinates calculated by the aberration sensitivity measurement system of the present embodiment on clipping coordinates.
[FIG. 23] A diagram showing an example of an image to which blur aberration presented by the aberration sensitivity measurement system of the present embodiment is applied.
[FIG. 24] A diagram showing an example of an aberration condition for blur aberration of the present embodiment.
[FIG. 25] A diagram showing an example of an aberration condition for distortion aberration of the present embodiment.
[FIG. 26] A diagram showing an example of a condition of an astigmatic axis of a left eye and an astigmatic axis of a right eye in a binocular distortion sensitivity test.
[FIG. 27] A diagram showing a modification example of an aberration condition for distortion aberration of the present embodiment.
[FIG. 28] A diagram showing a modification example of a condition of an astigmatic axis of a left eye and an astigmatic axis of a right eye in a binocular distortion sensitivity test.
[FIG. 29] A diagram showing an example of a measurement result by the aberration sensitivity measurement system of the present embodiment.
[FIG. 30] A diagram showing a first example of an analysis result by the aberration sensitivity measurement system of the present embodiment.
[FIG. 31] A diagram showing a second example of an analysis result by the aberration sensitivity measurement system of the present embodiment.
[FIG. 32] A diagram showing a third example of an analysis result by the aberration sensitivity measurement system of the present embodiment.
[FIG. 33] A diagram showing a fourth example of an analysis result by the aberration sensitivity measurement system of the present embodiment.
[FIG. 34] A diagram showing a first example of a calculation result of an angle distribution of a head rotation angle.
[FIG. 35] A diagram showing a second example of a calculation result of an angle distribution of a head rotation angle.
[FIG. 36] A diagram showing a first example of a calculation result of an angle distribution of a right/left eye rotation angle.
[FIG. 37] A diagram showing a second example of a calculation result of an angle distribution of a right/left eye rotation angle.
[FIG. 38] A diagram showing a first example of a calculation result of a binocular line-of-sight rotation width.
[FIG. 39] A diagram showing a second example of a calculation result of a binocular line-of-sight rotation width.
[FIG. 40] A diagram showing a first example of a calculation result of an average aberration addition level.
[FIG. 41] A diagram showing a second example of a calculation result of an average aberration addition level.
[FIG. 42] A diagram showing an example of an Euler angle in the virtual space
[FIG. 43] A diagram showing an example of change in line-of-sight angle in gazing steadily at a line-of-sight guide object.
[FIG. 44] A diagram showing an example of change in head angle in gazing steadily at the line-of-sight guide object.
[FIG. 45] A diagram showing an example of a result of composing the change in the line-of-sight angle and the change in the head angle.
[FIG. 46] A diagram showing a first example of an analysis result of a right and left sensitivity balance.
[FIG. 47] A diagram showing a second example of an analysis result of a right and left sensitivity balance.
[FIG. 48] A diagram showing an example of a correspondence relationship between an aberration amount and a line-of-sight range of a subject.
[FIG. 49] A diagram showing a first example of an analysis result of a line-of-sight usage ratio.
[FIG. 50] A diagram showing a second example of an analysis result of a line-of-sight usage ratio.
[FIG. 51] A diagram showing an example of a functional configuration of a spectacle lens ordering and order reception system of the present embodiment.
[FIG. 52] A diagram illustrating an example of a flow of an operation of the spectacle lens ordering and order reception system of the present embodiment.
[FIG. 53] A diagram illustrating an example of a flow of a detailed operation of the spectacle lens ordering and order reception system.
[FIG. 54] A diagram illustrating an example of a flow of a detailed operation of the spectacle lens ordering and order reception system.

### DESCRIPTION OF EMBODIMENTS

Hereinafter, an aberration sensitivity measurement system 5 of the present embodiment will be described with reference to the drawings.

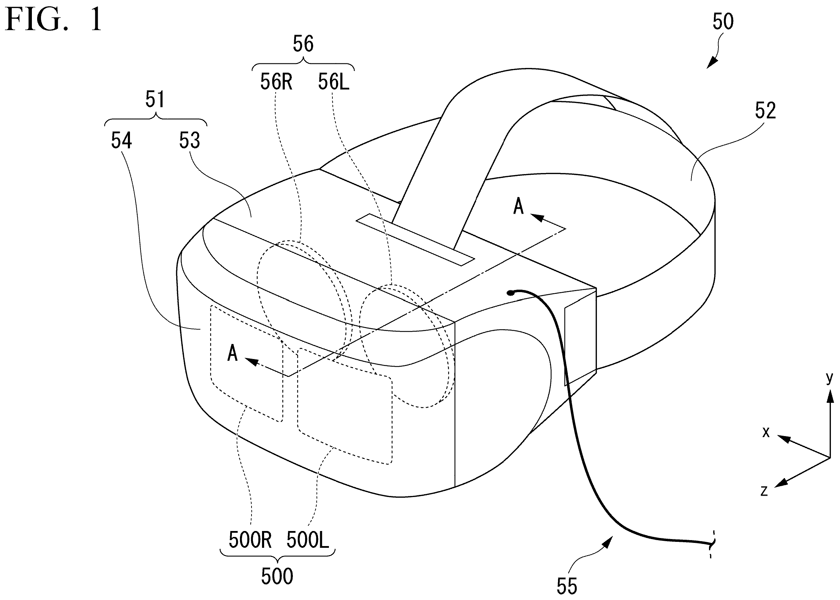

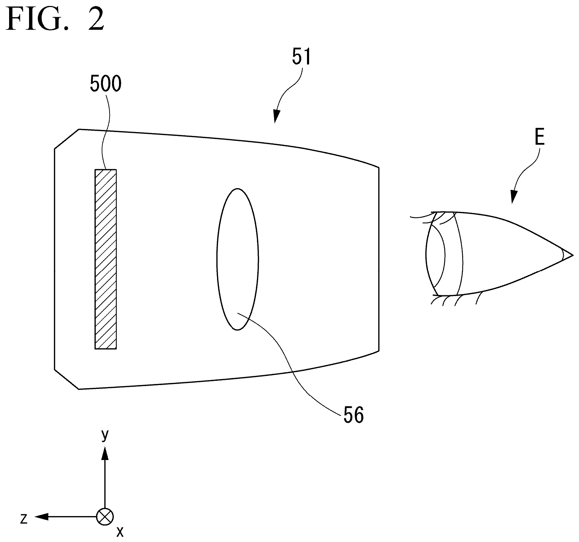

The aberration sensitivity measurement system 5 includes a terminal device 100 and a display device 50. A configuration of the display device 50 will be described with reference to FIGS. 1 and 2.

FIG. 1 is a perspective view showing an appearance of the display device 50 of the present embodiment. The display device 50 includes a main body 51 including a display screen and a support unit 52 that fixes the main body 51 to a head of a subject such that a position relationship between a face of the subject and the display device 50 is maintained. The main body 51 includes a lens holding unit 53 and a display screen holding unit 54 disposed in front of the lens holding unit 53.

The lens holding unit 53 holds image forming lenses 56L and 56R that form images of light from the display screen in a left eye and a right eye of the subject, respectively. The image forming lens includes one or a plurality of lenses. The display screen holding unit 54 will be described as a display unit 500.

The display unit 500 includes a left eye display unit 500L that displays an image 501L for a left eye to the left eye of the subject, and a right eye display unit 500R that displays an image 501R for a right eye to the right eye of the subject.

The display device 50 is connected to the terminal device 100 by a connection cable 55. Image information is transmitted from the terminal device 100 to the display device 50 via the connection cable 55. The display device 50 displays the image 501L for the left eye and the image 501R for the right eye on the display unit 500 on the basis of the image information transmitted from the terminal device 100. Further, various kinds of measurement information described below are transmitted from the display device 50 to the terminal device 100 via the connection cable 55. The terminal device 100 performs various arithmetic operations described below on the basis of the measurement information transmitted from the display device 50.

The display device 50 and the terminal device 100 may be connected by wireless communication, instead of the above-described wired communication (or in addition to the wired communication).

FIG. 2 is a diagram schematically showing an A-A cross section (FIG. 1) of the main body 51 of the display device 50. The display device 50 is disposed on the basis of a height of an eye E of the subject such that the eye E of the subject faces an image forming lens 56. Light emitted from the display unit 500 is transmitted through the image forming lens 56 and is incident on the eye E of the subject. The image forming lens 56 has positive refractive power, and accordingly, the subject can easily focus the eye E on the display unit 500 disposed in a head mounted display (HMD).

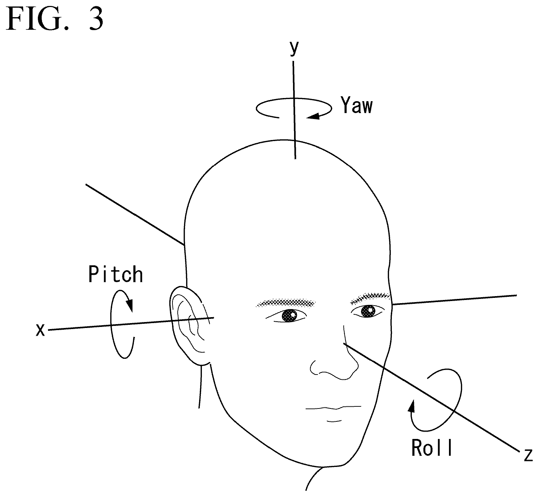

FIG. 3 is a diagram illustrating an Euler angle. In the case of indicating a direction of the head of the subject with the Euler angle, coordinate axes are determined as in the drawing. That is, in a local coordinate system in which a forward direction of the subject is a z axis, an upward direction is a y axis, and a right direction is an x axis, yaw becomes rotation (right-left) around the y axis, pitch becomes rotation (up-down) around the x axis, and roll becomes rotation around the z axis. In the case of indicating a direction of a line of sight of the subject with the Euler angle, a center of an xyz coordinate system is set as a rotation center position of the eye.

### [Functional Configuration of Aberration Sensitivity Measurement System]

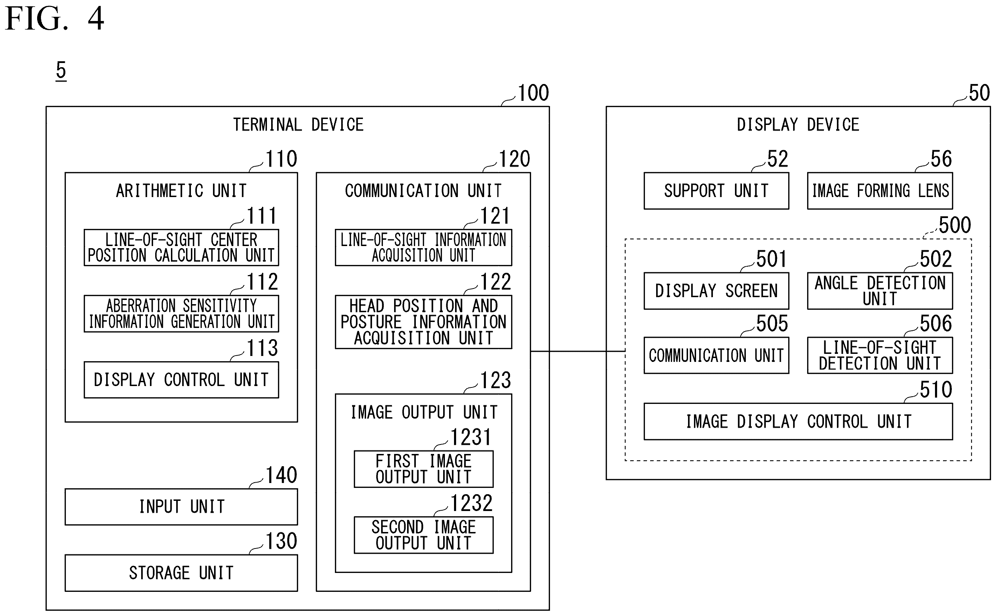

FIG. 4 is a diagram showing an example of a functional configuration of the aberration sensitivity measurement system 5 of the present embodiment. In the present embodiment, functions regarding image display other than the functions of the support unit 52, the image forming lens 56, and the like described above are provided in the display unit 500.

The display unit 500 includes a display screen 501, an angle detection unit 502, a communication unit 505, a line-of-sight detection unit 506, and an image display control unit 510.

The angle detection unit 502 includes an acceleration sensor, a geomagnetism sensor, and/or a gyro sensor, and detects values of an orientation, an angle, an angular velocity, and/or the like of the display unit 500. The image display control unit 510 described below calculates an angle at which the subject rotates the head or a direction in which the subject is facing, on the basis of the detected values.

The line-of-sight detection unit 506 includes a camera or the like that images the eye of the subject, and detects a line-of-sight direction of the left eye and a line-of-sight direction of the right eye of the subject individually from images of the right and left eyes of the subject.

The communication unit 505 includes a communication device for performing communication with the display device, and performs processing of acquiring an image for use in an aberration sensitivity examination that is provided from the terminal device 100 or transmitting line-of-sight information and the like.

The image display control unit 510 includes a processor such as a CPU, executes a program stored in a storage unit (not shown), and displays an image to which an aberration described below has been applied, on the display screen 501.

The terminal device 100 is, for example, a computer device, and includes an arithmetic unit 110, a communication unit 120, a storage unit 130, and an input unit 140.

The input unit 140 receives an input of a program screen on the display screen of the terminal device, and receives an input of various parameters for use in an aberration sensitivity examination or an operation of measurement.

The communication unit 120 performs communication with the communication unit 505 of the display device 50 on the basis of a predetermined communication protocol. The communication unit 120 includes, as functional units thereof, a line-of-sight information acquisition unit 121, a head position and posture information acquisition unit 122, and an image output unit 123.

The head position and posture information acquisition unit 122 acquires head position and posture information obtained by detecting movement of the head of the subject presented with at least one of the image 501L for the left eye and the image 501R for the right eye.

The line-of-sight information acquisition unit 121 acquires line-of-sight information obtained by detecting movement of the line of sight of the subject presented with at least one of the image 501L for the left eye and the image 501R for the right eye.

The image output unit 123 includes a first image output unit 1231 and a second image output unit 1232.

The first image output unit 1231 outputs image information of the image 501L for the left eye to the display screen 501 (left eye display unit) that presents an image to the left eye of the subject. In the image 501L for the left eye, a first aberration distribution that is an aberration distribution for the left eye can be set.

The second image output unit 1232 outputs image information of the image 501R for the right eye to the display screen 501 (right eye display unit) that presents an image to the right eye of the subject. In the image 501R for the right eye, a second aberration distribution that is an aberration distribution for the right eye can be set.

That is, the image output unit 123 can output the image 501L for the left eye and the image 501R for the right eye individually.

Both the image information of the image 501L for the left eye and the image information of the image 501R for the right eye are generated by the arithmetic unit 110.

The storage unit 130 includes a non-volatile storage medium, and stores various programs such as a program for image display for use in the aberration sensitivity examination or an image to be displayed.

The arithmetic unit 110 includes, for example, a central processing unit (CPU), operates on the basis of the programs and data stored in the storage unit 130, and provides various functions. The arithmetic unit 110 includes, as functional units thereof, a line-of-sight center position calculation unit 111, an aberration sensitivity information generation unit 112, and a display control unit 113.

The line-of-sight center position calculation unit 111 calculates a line-of-sight center position on the basis of the line-of-sight information of the subject.

An aberration distribution that is set in the image 501L for the left eye, that is, the aberration distribution for the left eye (first aberration distribution) is an aberration distribution (function) with a line-of-sight center position of the left eye of the subject as an origin position.

Here, the aberration distribution (function) means an aberration distribution that is indicated by a function defining an aberration.

An aberration distribution that is set in the image 501R for the right eye, that is, the aberration distribution for the right eye (second aberration distribution) is an aberration distribution based on a line-of-sight center position of the right eye of the subject.

The line-of-sight center position calculation unit 111 calculates the line-of-sight center position on the basis of a frequency distribution of the line of sight indicated by the line-of-sight information of the subject. For example, the line-of-sight center position calculation unit 111 calculates the line-of-sight center position on the basis of a predetermined range (for example, a range of 90% at the center excluding an upper limit of 10% and a lower limit of 10%) of the frequency distribution of the line of sight indicated by the line-of-sight information of the subject.

The aberration sensitivity information generation unit 112 generates aberration sensitivity information that is information regarding sensitivity of the subject to aberration, on the basis of the acquired line-of-sight information and the aberration distribution.

In a case where the terminal device 100 includes the head position and posture information acquisition unit 122, the aberration sensitivity information generation unit 112 generates aberration sensitivity information on the basis of the acquired head position and posture information.

The display control unit 113 generates an aberration image obtained by applying various aberrations to an image with no aberration. The display control unit 113 outputs the generated aberration image to the image output unit 123.

The first image output unit 1231 and the second image output unit 1232 may sequentially output a plurality of pieces of image information having different distribution forms of aberration. In this case, the aberration sensitivity information generation unit 112 generates aberration sensitivity information for each of the distribution forms of aberration that are sequentially output.

The distribution forms of aberration that is applied to the image with no aberration by the image output unit 123 may include a distribution form (first distribution form) in which a degree of aberration is different between the right and left eyes.

The distribution forms of aberration that is applied to the image with no aberration by the image output unit 123 may include a distribution form (second distribution form) in which a degree of aberration is uniform within a display range.

The distribution forms of aberration that is applied to the image with no aberration by the image output unit 123 may include a distribution form (third distribution form) in which a degree of aberration at a center position of the line of sight of the subject is set to be smaller than a degree of aberration in an edge portion of the display range.

In the above description, "the distribution forms of aberration that is applied to the image with no aberration by the image output unit 123" may be read as "a distribution form of aberration of an image that is output from the image output unit 123, which is the distribution form of aberration applied to the image with no aberration by the display control unit 113".

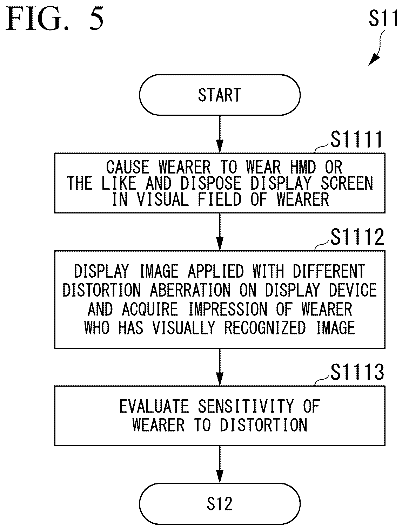

FIG. 5 is a diagram illustrating an example of a flow of an operation of sensitivity evaluation by the aberration sensitivity measurement system 5 of the present embodiment. The aberration sensitivity measurement system 5 sequentially presents images applied with aberration distributions to the subject and measures aberration sensitivity of the subject. The images applied with the aberration distributions include an image applied with distortion aberration and an image applied with blur aberration. In the following description, a case where an image applied with distortion aberration is presented as an example of an image applied with an aberration distribution will be described, an image applied with blur aberration is also presented similarly. Further, an image applied with distortion aberration and blur aberration in combination may be presented to the subject.

In Step S1111, a user (for example, a person in charge of examination at an optician's store) of the terminal device 100 causes the subject to wear the display device 50 such as an HMD and disposes the display screen 501 in a visual field of the subject. After Step S1111 ends, the process proceeds to Step S1112.

In Step S1112, the person in charge of examination sequentially displays images Y (hereinafter, also referred to as images Y applied with distortion aberration or distortion aberration-applied images Y, or simply referred to as images Y) obtained by applying a plurality of distortion aberrations including different aberrations to an image with no aberration on the display screen 501, causes the subject to visually recognize the distortion aberration-applied image Y, and acquires an impression of the subject who has visually recognized the distortion aberration-applied image Y. The person in charge of examination displays the distortion aberration-applied image Y in regions of the display screen 501 corresponding to lateral portions La1 and La2 (not shown) of a progressive refractive power lens.

Here, the aberration sensitivity measurement system 5 outputs the image 501L for the left eye and the image 501R for the right eye from the terminal device 100 to the display device 50 on the basis of an operation of the person in charge of examination. The display device 50 presents the image 501L for the left eye and the image 501R for the right eye output from the terminal device 100 to the subject.

The person in charge of examination fixes a distortion direction of the distortion aberration to be displayed, increases a degree of distortion, and specifies a distortion direction or (and) a degree of distortion of the distortion aberration for which the subject replies that the distortion aberration-applied image Y (that is, the image Y after the distortion aberration is changed) is unacceptable.

Here, the aberration sensitivity measurement system 5 sets a degree of distortion on the basis of a setting operation of a degree of distortion of the person in charge of examination and generates the image 501L for the left eye and the image 501R for the right eye with the generated degree of distortion.

In addition, the person in charge of examination fixes a distortion direction in a state in which a distortion direction is changed, increases a degree of distortion, and specifies the distortion aberration-applied image Y for which the subject replies that the distortion aberration-applied image Y is unacceptable. In this way, a plurality of distortion aberration-applied images Y having different degrees of distortion for one or a plurality of distortion directions are displayed. After Step S1112 ends, the process proceeds to Step S1113.

An order and the number of repetitions in which the distortion aberration-applied images Y having different degrees of distortion are presented are not limited.

In Step S1113, the person in charge of examination evaluates sensitivity to distortion in a visual field of the subject who has visually recognized the distortion aberration-applied image Y. The person in charge of examination converts the sensitivity of the subject to distortion on the basis of the reply from the subject who has visually recognized the distortion aberration-applied image Y, obtained in Step S1112 into a numerical value according to a criterion determined in advance and records the numerical value. For example, in the case of increasing the degree of distortion of the distortion aberration-applied image Y as described above until the subject replies that the distortion aberration-applied image Y is unacceptable, a degree of distortion for which the subject initially replies that the distortion aberration-applied image Y is unacceptable or a degree of distortion that is a limit acceptable to the subject is acquired as a parameter (hereinafter, called a sensitivity parameter) indicating sensitivity of the subject to distortion. That is, the sensitivity parameter is determined on the basis of the above-described specified distortion aberration-applied image Y.

### [Measurement Scene]

Next, a measurement scene by the aberration sensitivity measurement system 5 will be described. The measurement scene is a type of a virtual space VS that is presented to the subject via the display device 50 by the aberration sensitivity measurement system 5. In the following description, the measurement scene is also simply referred to as a "scene".

### [Measurement Scene (1) Measurement of Blur Sensitivity]

A measurement scene in measuring blur sensitivity among measurements of aberration sensitivity will be described. In the measurement scene of blur sensitivity, as an example, there are a far vision scene (for a far distance vision scene; the same applies hereafter.), an intermediate distance vision scene, a near vision scene (or a near distance vision scene), and the like.

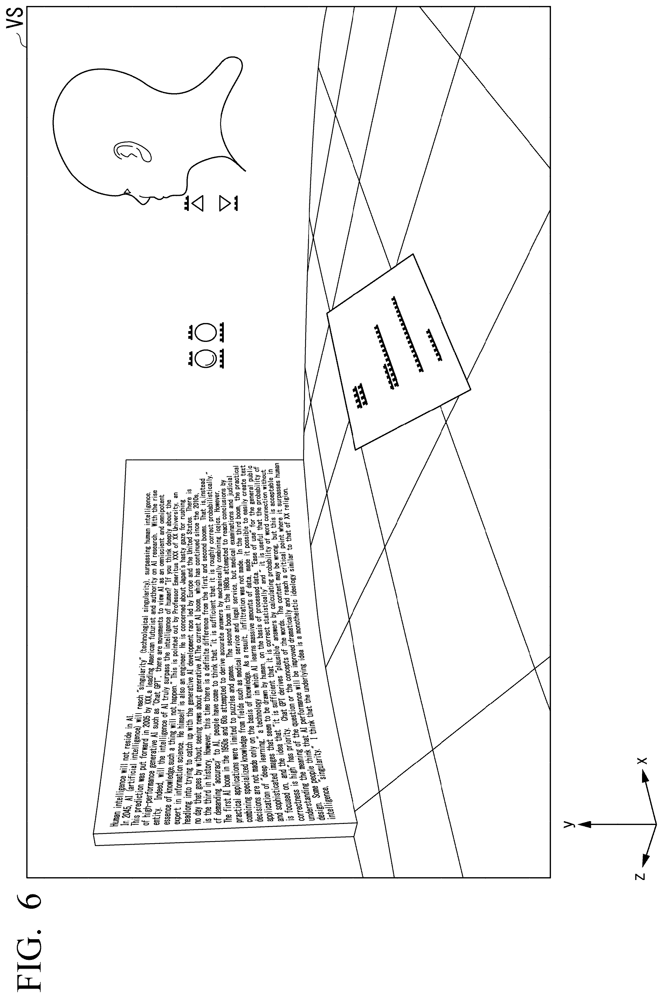

FIG. 6 is a diagram showing a first example of a measurement scene in the virtual space VS of the present embodiment. In the drawing, an example of a far vision scene is shown. The far vision scene is a scene for measuring aberration sensitivity (for example, blur sensitivity) for far vision. In the far vision scene, a virtual display is disposed at a position separated from the subject by 3 [m] in a depth direction (+z direction) in the virtual space VS. A size of the virtual display is set to a range in which the subject can visually recognize the virtual display only by changing a line-of-sight direction without shaking the head in a right-left direction (that is, without rotating the head in a yaw direction). In this example, a linear function is applied to a blur aberration function.

On the virtual display in the virtual space VS, as an example, horizontally written text is displayed. The aberration sensitivity measurement system 5 presents an image of horizontally written text to the right and left eyes of the subject individually.

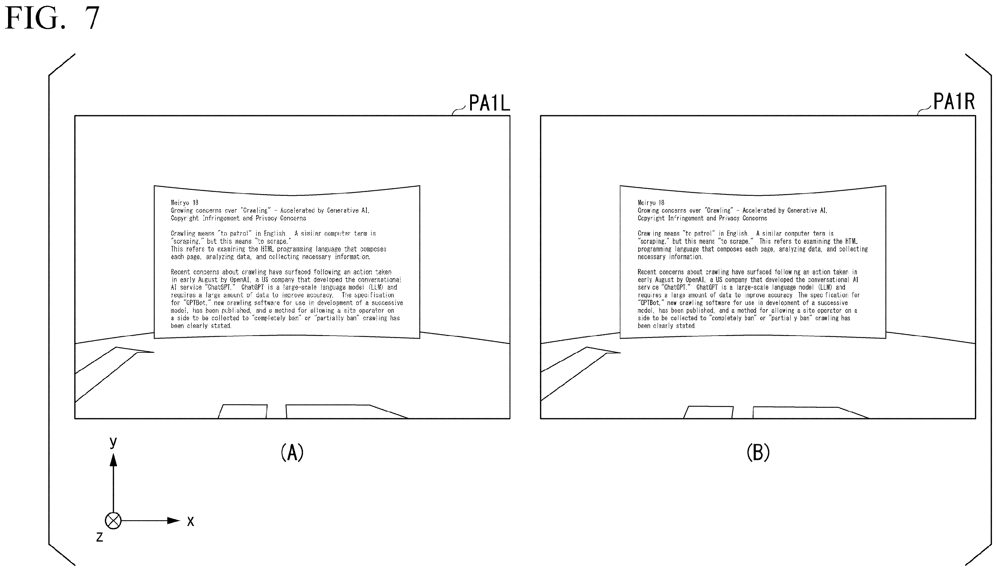

FIG. 7 is a diagram showing an example of images PA1 applied with blur aberration that are presented by the aberration sensitivity measurement system 5 of the present embodiment. In (A) of the drawing, an image PA1L applied with blur aberration for a left eye is shown. In (B) of the drawing, an image PA1R applied with blur aberration for a right eye is shown. The subject recognizes that horizontally written text is disposed at the position separated from the subject by 3 [m] in the depth direction (+z direction) in the virtual space VS by visually recognizing the image PA1L applied with blur aberration for the left eye and the image PA1R applied with blur aberration for the right eye with the left eye and the right eye, respectively.

The aberration sensitivity measurement system 5 can individually set a state of blur of the image PA1L applied with blur aberration for the left eye and a state of blur of the image PA1R applied with blur aberration for the right eye.

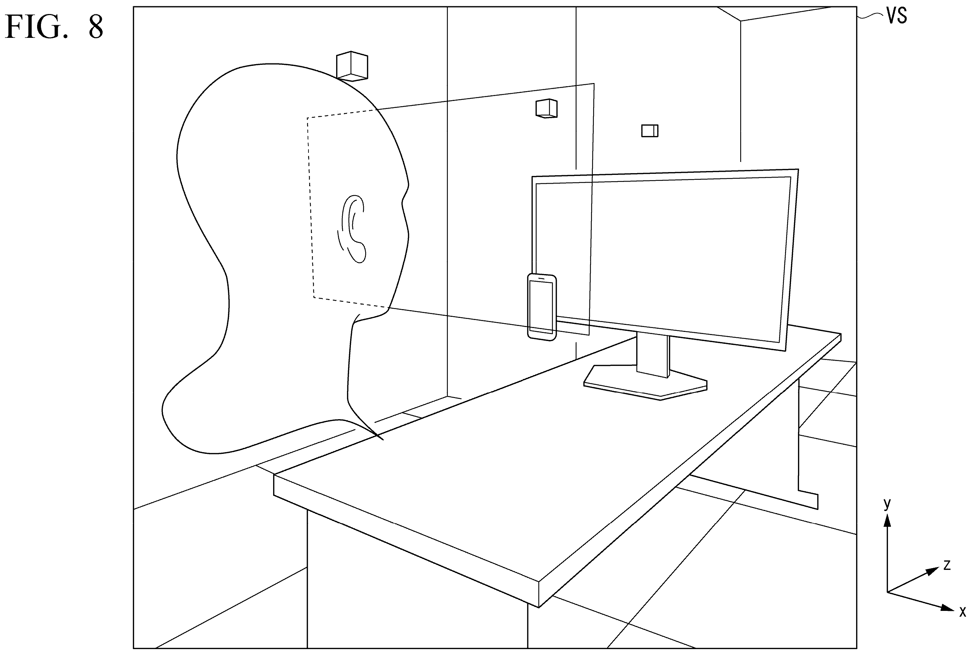

FIG. 8 is a diagram showing a second example of a measurement scene in the virtual space VS of the present embodiment. In the drawing, an example of an intermediate distance vision scene is shown. The intermediate distance vision scene is a scene for measuring aberration sensitivity (for example, blur sensitivity) at an intermediate distance that is a distance between far vision and near vision. In the intermediate distance vision scene, a virtual display is disposed at a position separated from the subject by 1 [m] in the depth direction (+z direction) in the virtual space VS.

In this example, a constant function is applied to a blur aberration function.

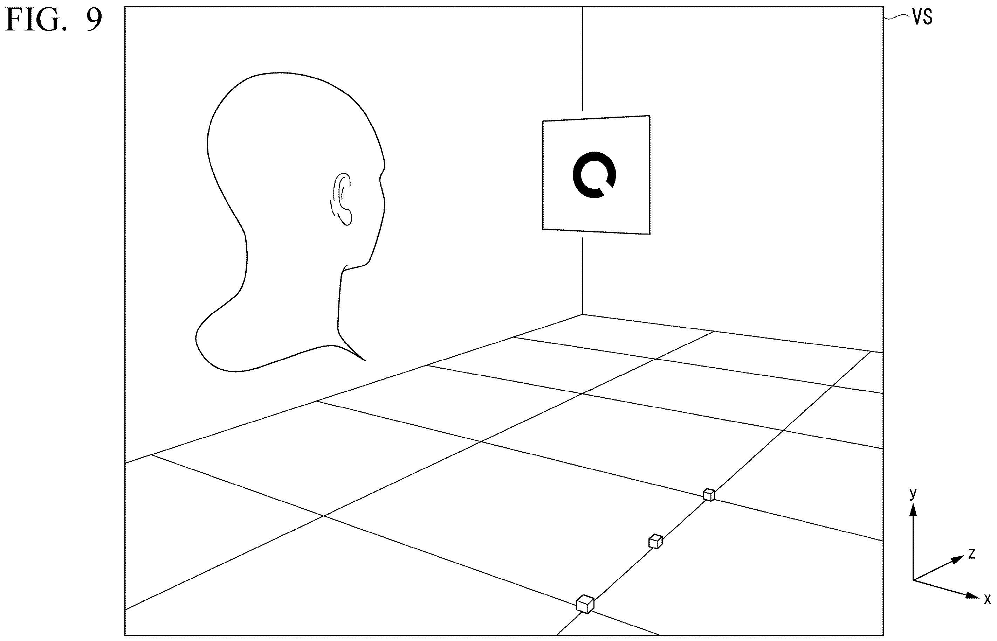

FIG. 9 is a diagram showing a third example of a measurement scene in the virtual space VS of the present embodiment. In the drawing, a modification example of an intermediate distance vision scene is shown. In this modification example, a figure (for example, a Landolt ring) for virtual visual acuity (for example, spatial resolution) measurement is disposed at a position separated from the subject by 1 [m] in the depth direction (+z direction) in the virtual space VS.

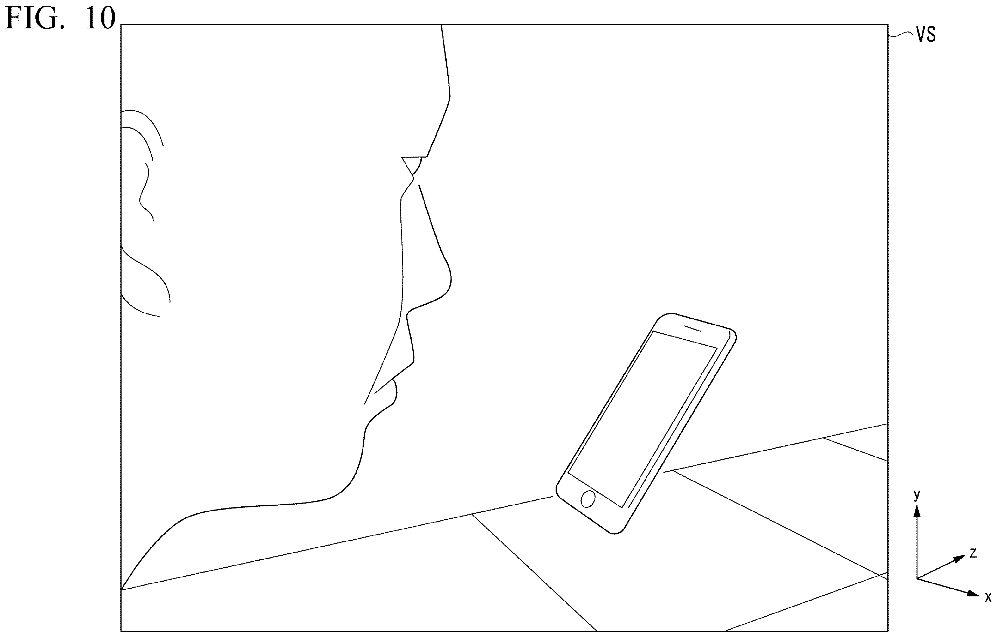

FIG. 10 is a diagram showing a fourth example of a measurement scene in the virtual space VS of the present embodiment. In the drawing, an example of a near vision scene is shown. The near vision scene is a scene for measuring aberration sensitivity (for example, blur sensitivity) at a near distance. In the near vision scene, an object such as a virtual display device, a newspaper, or a book is disposed at a position within about 1.0 m from the subject in the virtual space VS.

In the measurement of blur sensitivity, it is not necessary to guide the line of sight of the subject. For this reason, the aberration sensitivity measurement system 5 does not display a guide object PG for guiding the line of sight of the subject in the measurement scene of blur sensitivity.

### [Measurement Scene (2) Measurement of Distortion Sensitivity]

A measurement scene in measuring distortion sensitivity among measurements of aberration sensitivity will be described. In the measurement of distortion sensitivity, sensitivity of the subject to distortion in a state in which the line of sight or the head of the subject is rotated around the yaw axis is measured. For this reason, unlike the measurement scene of blur sensitivity described above, in the measurement scene of distortion sensitivity, the aberration sensitivity measurement system 5 disposes the guide object PG such as a sphere for guiding the line of sight of the subject in the scene.

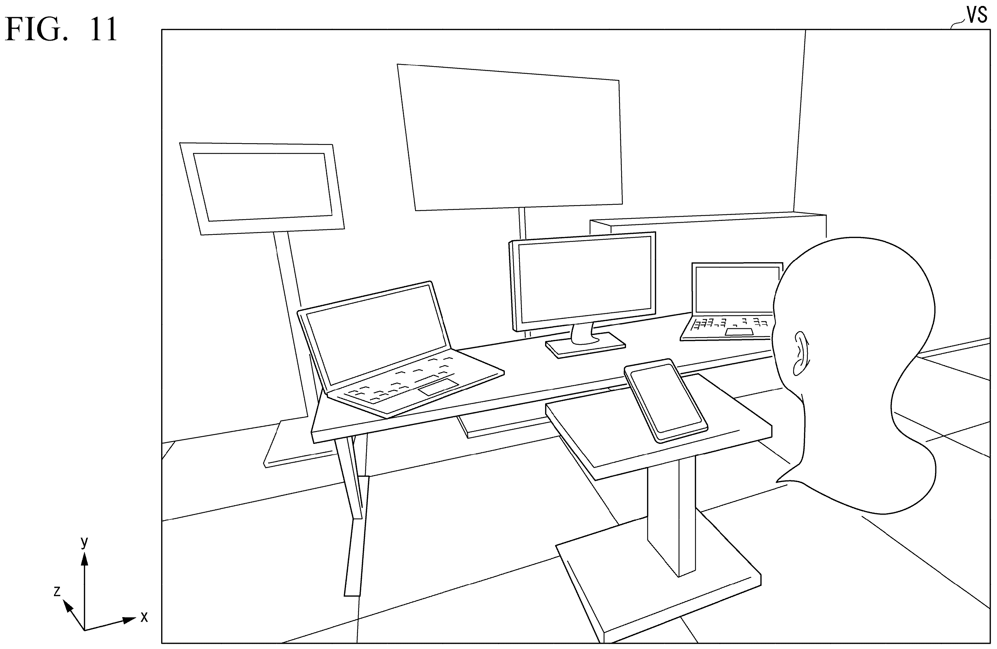

FIG. 11 is a diagram showing a fifth example of a measurement scene in the virtual space VS of the present embodiment. In the drawing, an example of a scene for distortion sensitivity measurement is shown. The scene for distortion sensitivity measurement is, for example, a scene that simulates a status of a desk during working. In the distortion sensitivity measurement scene, images of a personal computer, a display, and a tablet are disposed at an intermediate distance (for example, a distance within about 2.0 to 3.0 m from the subject) in the virtual space VS.

In the distortion sensitivity measurement scene, the aberration sensitivity measurement system 5 presents an image PA2 applied with distortion aberration to the subject in the virtual space VS. For example, in the image PA2 applied with distortion aberration, a movement amount of a position of each point of an object due to distortion is given to be different at each position of a frustum with a viewpoint as a vertex.

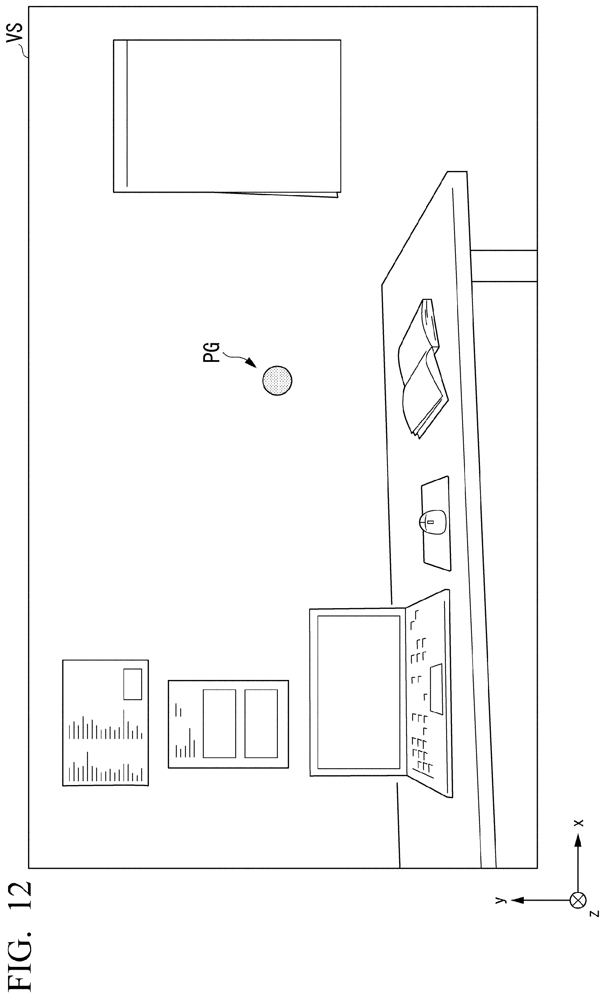

FIG. 12 is a diagram showing an example of a line-of-sight guide in the virtual space VS of the present embodiment. In the drawing, as an example of the line-of-sight guide, a guide object PG (that is an example of a line-of-sight guide object and may have any shape) that is a virtual sphere floating in the virtual space VS is shown. The aberration sensitivity measurement system 5 moves the guide object right and left (x-axis direction), up and down (y-axis direction), and front and back (z-axis direction) in the virtual space VS. The subject traces the guide object PG that moves right and left, up and down, and front and back, with line-of-sight movement or head movement. The aberration sensitivity measurement system 5 can obtain a measurement result in a case where the subject views the guide object PG that moves right and left, up and down, and front and back, with distortion.

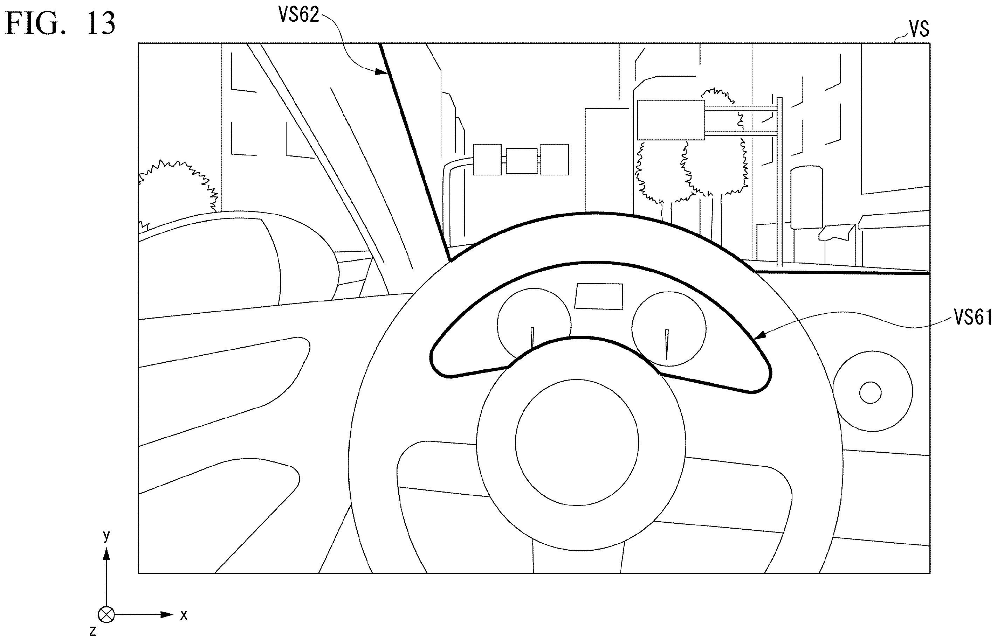

FIG. 13 is a diagram showing a sixth example of a measurement scene in the virtual space VS of the present embodiment. In the drawing, as an example of a scene for distortion sensitivity measurement, a status viewed from the vehicle inside of an automobile that is driven by the subject is shown. In the scene of the vehicle inside shown in the drawing, line-of-sight data when the subject views a near distance (operation panel portion VS61) and a far distance (forward visual field portion VS62 such as a sign or a street) with states of distortion and blur aberration can be obtained.

### [Type of Aberration that is Presented by Aberration Sensitivity Measurement System]

The aberration sensitivity measurement system 5 can present the virtual space VS including various types of aberrations. For example, in the aberrations of the spectacle lens, there are blur and distortion. In this case, the aberration sensitivity measurement system 5 presents blur, distortion, and a combination of blur and distortion as aberrations to the subject.

### (1) Blur

Blur in the present embodiment is a phenomenon in which an optical wavefront emitted from one point on an object does not converge to one point at a retina position and spreads out. The aberration sensitivity measurement system 5 reproduces a phenomenon of blur in the virtual space VS through image processing.

### (2) Distortion

Distortion in the present embodiment is a phenomenon in which a path of a light beam is changed by the spectacle lens and a shape of an object appears to be changed from an original shape. The aberration sensitivity measurement system 5 reproduces a phenomenon of distortion in the virtual space VS through image processing. The aberration sensitivity measurement system 5 applies a value or the like calculated from any aberration function f(x,y) or the design data of the spectacle lens as an amount of distortion (for example, an amount of change in position where an object appears, due to change in path of a light beam) to reproduce distortion in the virtual space VS.

For the above-described aberration such as blur or distortion, an aberration amount according to a distance of an object can be applied by calculating a distribution of aberration in a viewing frustum in advance.

### [Definition of Aberration Function]

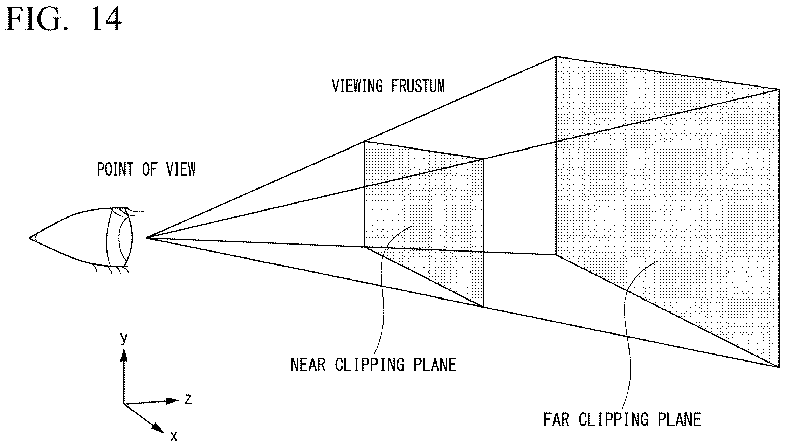

FIG. 14 is a diagram showing an example of a clipping plane of a viewing frustum. The aberration sensitivity measurement system 5 generates an image obtained by projecting a measurement scene onto a near clipping plane.

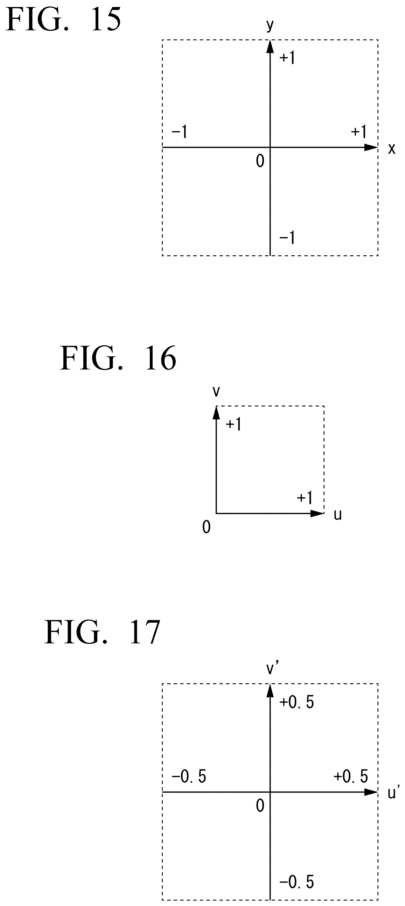

FIG. 15 is a diagram showing an example of a coordinate system of a near clipping plane. The near clipping plane is a coordinate system in which the x axis and the y axis are normalized in a range of -1 to +1. In the aberration function f(x,y), a variable x and a variable y are defined in a range of -1 to +1.

In general, aberration is defined by a function with a position (xg, yg) or an angle (θh, θv) as a variable in a three-dimensional space.

FIG. 16 is a diagram showing an example of a texture coordinate system. The texture coordinate system is a coordinate system that is used to represent coordinates of image data. In a case where image data is defined in the texture coordinate system (UV coordinate system), in an aberration function f(u,v), a variable u and a variable v are defined in a range of 0 to 1.

The aberration sensitivity measurement system 5 performs image processing with the aberration function in a coordinate system (that is, an image processing coordinate system) with a center position of the texture coordinate system as an origin.

FIG. 17 is a diagram showing an example of the image processing coordinate system. An aberration function specifies an aberration value at each in-plane position in an image processing coordinate system (U'V' coordinate system) shown in the drawing.

### [Coordinate Conversion of Clipping Plane]

A procedure for converting a function with a position or an angle as a variable into an image processing coordinate system will be described.

A vertical direction viewing angle of the viewing frustum is denoted by fovy, a horizontal direction viewing angle is denoted by fovx, and a distance from a viewpoint to a clipping plane is denoted by d.

### [(1) Conversion from angle into normalization coordinates of clipping plane]

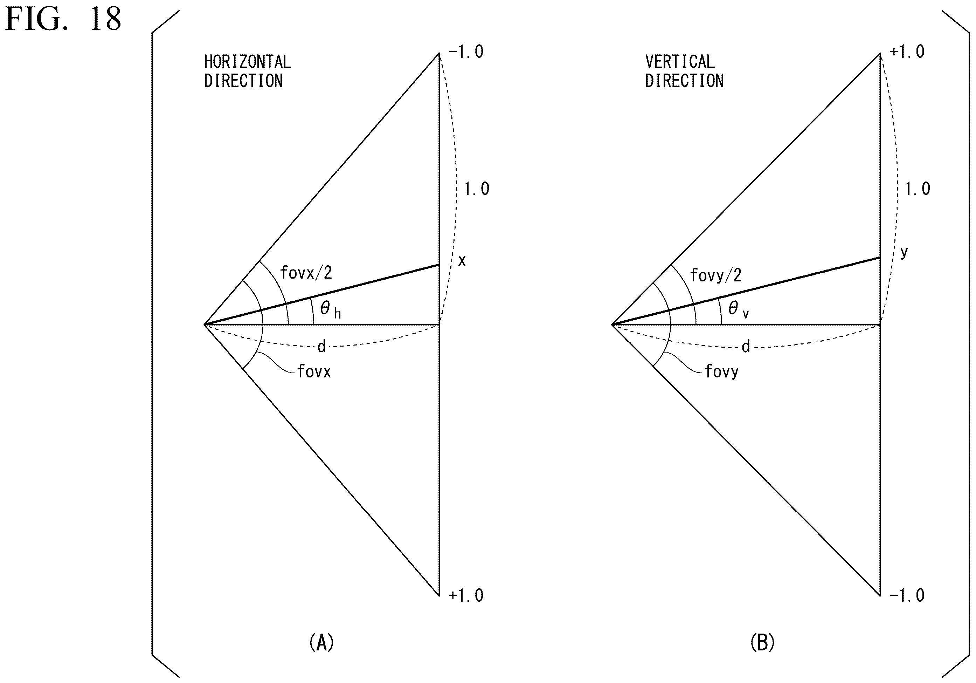

FIG. 18 is a diagram showing an example of conversion from an angle into normalization coordinates of clipping plane. In (A) of the drawing, an example of conversion with respect to the horizontal direction is shown. In (B) of the drawing, an example of conversion with respect to the vertical direction is shown. The angle θh in the horizontal direction and the angle θv in the vertical direction are converted into normalization coordinate value (x,y) on the basis of Expressions (1) and (2).
[Equation 1] $x = d tan {θ}_{h} = \frac{tan {θ}_{h}}{tan \left(\frac{fovx}{2}\right)}$ (1)
[Equation 2] $y = d tan {θ}_{v} = \frac{tan {θ}_{v}}{tan \left(\frac{fovy}{2}\right)}$ (2)

### [(2) Conversion from normalization coordinates of clipping plane into angle]

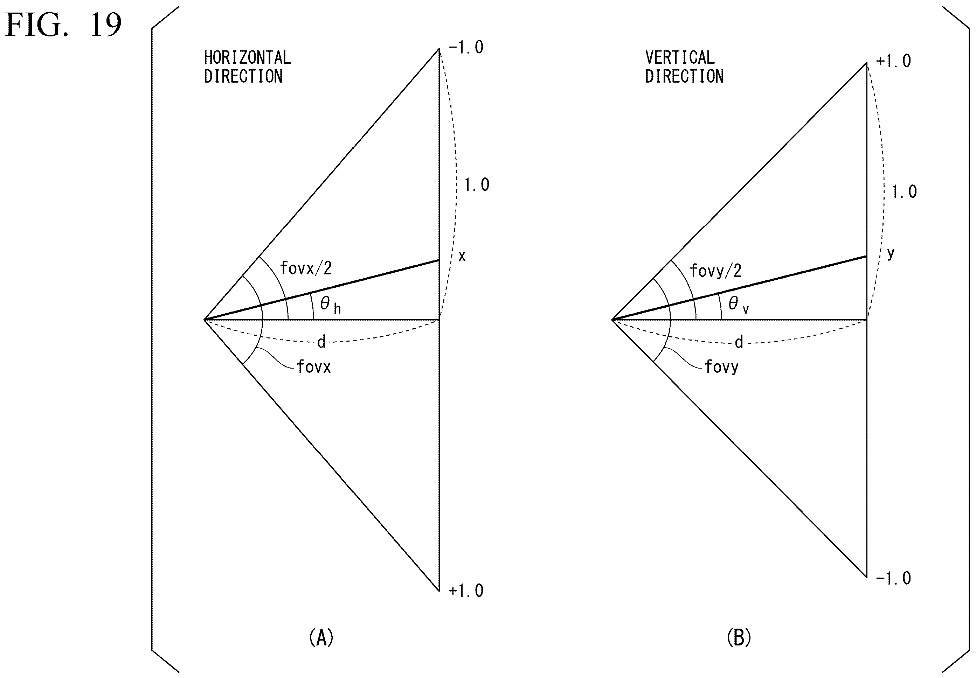

FIG. 19 is a diagram showing an example of conversion from normalization coordinates of a clipping plane into an angle. In (A) of the drawing, an example of conversion with respect to the horizontal direction is shown. In (B) of the drawing, an example of conversion with respect to the vertical direction is shown. The normalization coordinate value (x,y) are converted into the angle θh in the horizontal direction and the angle θv in the vertical direction on the basis of Expression (3) and Expression (4). The expressions are used in a case of inversely calculating an angle from a measurement result of clipping coordinates, not the conversion of the aberration function.
[Equation 3] ${θ}_{h} = {tan}^{- 1} \frac{x}{d} = {tan}^{- 1} \left(x tan\left(\frac{fovx}{2}\right)\right)$ (3)
[Equation 4] ${θ}_{v} = {tan}^{- 1} \frac{y}{d} = {tan}^{- 1} \left(y tan\left(\frac{fovy}{2}\right)\right)$ (4)

### [(3) Conversion from position into normalization coordinates of clipping plane]

The number of pixels of an image is set to w × h. In regard to a physical size of an image, when a distance to an object is denoted by dobj, a physical size of a plane at the distance dobj is W × H and is indicated by Expression (5).
[Equation 5] $\left[\begin{matrix}W = 2 ⋅ {d}_{obj} ⋅ tan \frac{fovx}{2} \\ H = 2 ⋅ {d}_{obj} ⋅ tan \frac{fovy}{2}\end{matrix}\right.$ (5)

A physical size per unit pixel is indicated by Expression (6).
[Equation 6] $\left[\begin{matrix}{u}_{W} = \frac{W}{w} \\ {u}_{h} = \frac{H}{h}\end{matrix}\right.$ (6)

The normalization coordinates (x,y) of the clipping plane from the position (xg, yg) are indicated by Expression (7).
[Equation 7] $\left[\begin{matrix}x = \frac{2 {x}_{g}}{W} \\ y = \frac{2 {y}_{g}}{H}\end{matrix}\right.$ (7)

### [(4) Conversion from clipping coordinates into texture coordinates]

Conversion from the normalization coordinates (x,y) of the clipping plane into a texture UV coordinate system is indicated by Expression (8).
[Equation 8] $\left[\begin{matrix}u = 0.5 x + 0.5 \\ v = 0.5 y + 0.5\end{matrix}\right.$ (8)

### [(5) Conversion from texture UV coordinate system into image processing coordinate system]

Conversion from the texture UV coordinate system into the image processing coordinate system is indicated by Expression (9).
[Equation 9] $\left[\begin{matrix}u ′ = u - 0.5 \\ v ′ = v - 0.5\end{matrix}\right.$ (9)

### [Blur Aberration Function]

A blur aberration function defines a size of a circle or an ellipse, which is a range of blur, for each position or angle. The physical size W × H of the plane and the size of the circle or the ellipse that is the range of blur can be converted into a size of a blur range in the image processing coordinate system. A shape of an aberration function is a given value and is indicated by Expression (10).
[Equation 10] $B \left(x, y\right) = const$ (10)

The blur aberration function can also be indicated by Expression (11) that is a linear function with a and b as any constants and xo and yo as center position offset amounts.
[Equation 11] $\left[\begin{matrix}B \left(x, y\right) = a \left|x-{x}_{o}\right| + b \\ B \left(x, y\right) = a \left|y-{y}_{o}\right| + b\end{matrix}\right.$ (11)

Here, in a case where a distance r from a center is expressed as Expression (12), the blur aberration function can also be indicated by an expression format of Expression (13).
[Equation 12] $r = \sqrt{{x}^{2} + {y}^{2}}$ (12)
[Equation 13] $B \left(x, y\right) = A r + b$ (13)

The aberration sensitivity measurement system 5 can also have the size of the blur range calculated by light beam tracing on the spectacle lens as a table.

### [Distortion Aberration Function]

A distortion aberration function defines an amount of displacement [Δx,Δy] in which a visual object is shifted, with distortion of the spectacle lens for each position or angle. A shape of the aberration function is indicated by Expression (15) with a as any constant and xo and yo as center position offset amounts in a case where the distance r from the center is indicated by Expression (14).
[Equation 14] $\left[\begin{matrix}{r}^{2} = {\left(x-{x}_{o}\right)}^{2} + {\left(y-{y}_{o}\right)}^{2} \\ r = \sqrt{{\left(x-{x}_{o}\right)}^{2} + {\left(y-{y}_{o}\right)}^{2}}\end{matrix}\right.$ (14)
[Equation 15] $D \left(x, y\right) = 1 + {r}^{2} + ar$ (15)

The aberration sensitivity measurement system 5 can also have the amount of displacement [Δx,Δy] calculated by light beam tracing on the spectacle lens as a table.

### [Measurement of Center Position of Aberration Function]

The aberration sensitivity measurement system 5 obtains a center of an aberration distribution in advance according to the following procedure when any aberration distribution is applied through image processing.

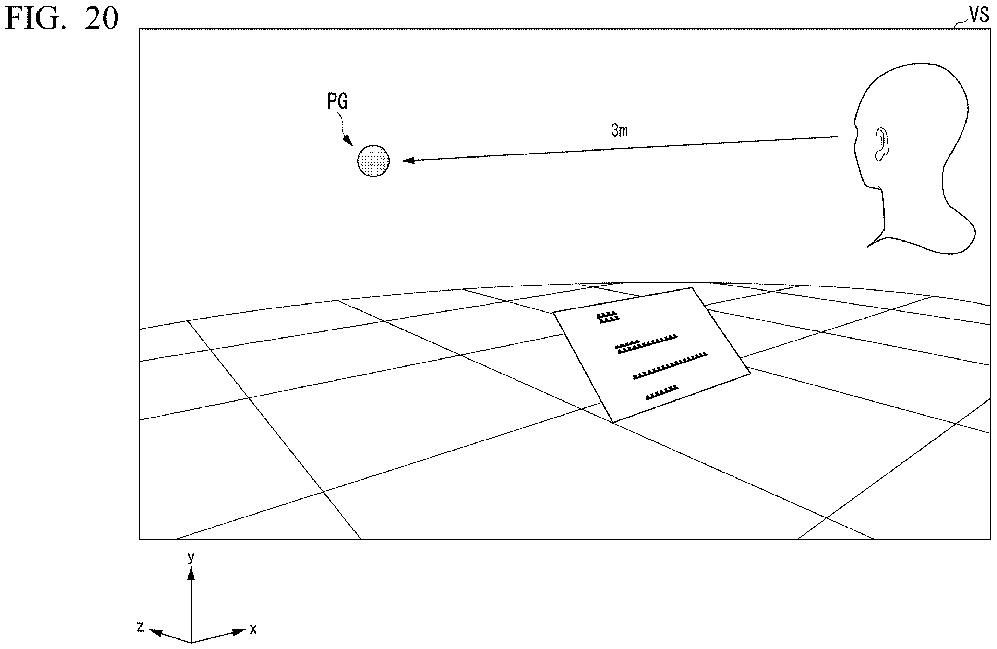

FIG. 20 is a diagram showing an example of the virtual space VS in obtaining a center of an aberration distribution in the present embodiment. The aberration sensitivity measurement system 5 displays the guide object PG (or also referred to as a line-of-sight guide object) in the virtual space VS and gets the subject fixate the guide object PG for about 15 seconds. In this case, the aberration sensitivity measurement system 5 does not change the position of the guide object PG in the virtual space VS.

The aberration sensitivity measurement system 5 detects a direction of the line of sight of the subject. The aberration sensitivity measurement system 5 calculates an intersection position of the direction of the line of sight of the subject and the near clipping plane of the viewing frustum.

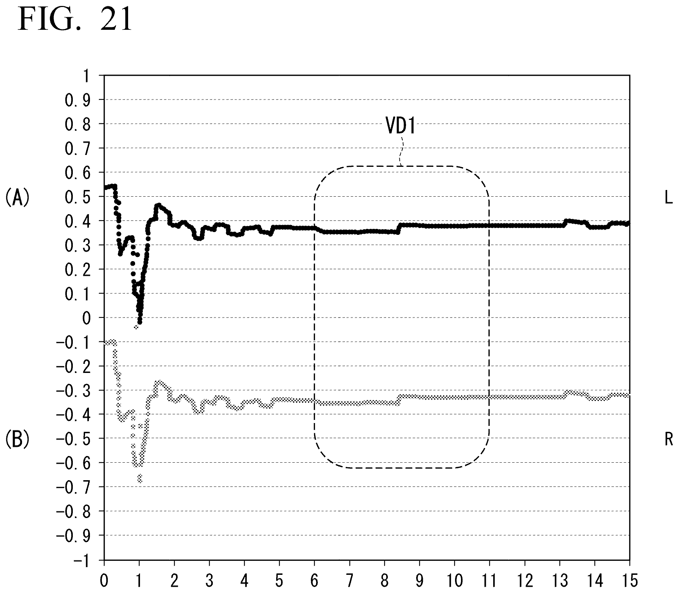

FIG. 21 is an example of change with time in intersection position of a direction of the line of sight of the subject and the near clipping plane of the viewing frustum. In the drawing, among coordinates (xy) indicating the intersection position of the direction of the line of sight of the subject and the near clipping plane of the viewing frustum, a coordinate of the x axis is illustrated. (A) of the drawing shows coordinates for the left eye of the subject. (B) of the drawing shows coordinates for the left eye of the subject.

As described, in obtaining the center of the aberration distribution, the aberration sensitivity measurement system 5 does not change the position of the guide object PG in the virtual space VS. On the other hand, a time is required depending on the subject until the subject matches the line of sight with the position of the guide object PG, and maintains the line of sight at that position (that is, until the subject fixates the guide object PG).

The aberration sensitivity measurement system 5 employs, as valid coordinate data VD1, coordinate data for five seconds from six seconds to 11 seconds after the start without employing coordinate data for about first six seconds after the guide object PG is displayed, from among coordinate data of the intersection position for about 15 seconds during which the subject is fixating the guide object PG.

In addition, the aberration sensitivity measurement system 5 rearranges coordinates in each of the x-axis direction and the y-axis direction in an ascending order (or a descending order) for the acquired valid coordinate data VD1. That is, the aberration sensitivity measurement system 5 converts coordinates of a line-of-sight center into a histogram in which the valid coordinate data VD1 acquired on the time axis is arranged in an order according to a distance from a center of the coordinates.

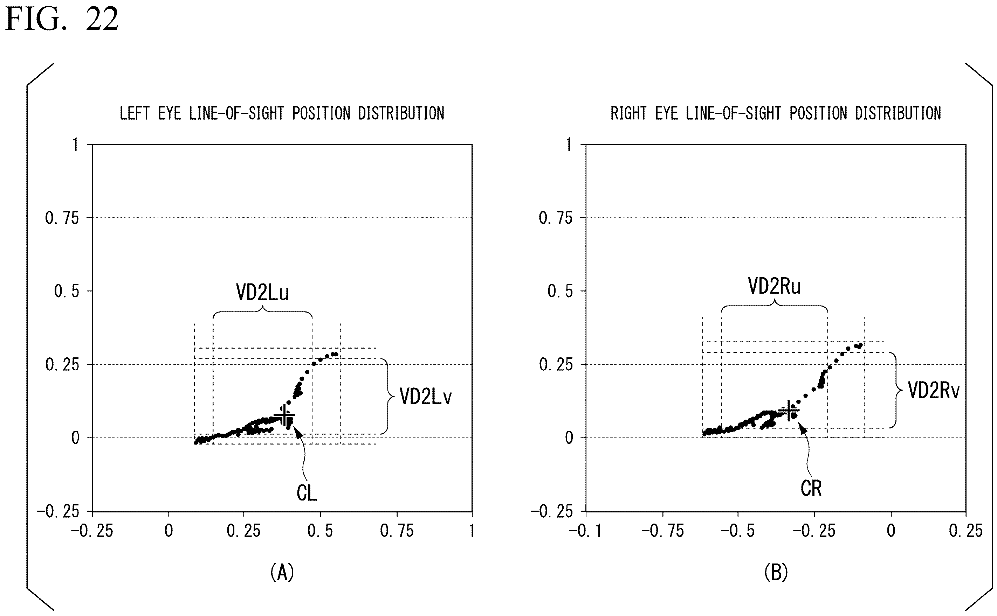

FIG. 22 is an example of a distribution of line-of-sight center coordinates calculated by the aberration sensitivity measurement system 5 of the present embodiment on clipping coordinates. (A) of the drawing shows a distribution of line-of-sight center coordinates of the left eye of the subject. (B) of the drawing shows a distribution of line-of-sight center coordinates of the right eye of the subject.

The aberration sensitivity measurement system 5 employs, as valid coordinate data VD2, coordinate data in a range of 90% on a center side in a coordinate axis direction without employing coordinate data of 5% on each of upper and lower sides in the coordinate axis direction in a distribution of line-of-sight center coordinates for each of x and y coordinates.

In an example shown in the drawing, the aberration sensitivity measurement system 5 employs, as valid coordinate data VD2L of the left eye of the subject, coordinate data included in valid coordinate data VD2Lx in the x-axis direction and valid coordinate data VD2Ly in the y-axis direction of the left eye of the subject. Further, the aberration sensitivity measurement system 5 employs, as valid coordinate data VD2R of the right eye of the subject, coordinate data included in valid coordinate data VD2Rx in the x-axis direction and valid coordinate data VD2Ry in the y-axis direction of the right eye of the subject.

A shake width of the line of sight may become large according to the subject in matching the line of sight with the position of the guide object PG. When the shake width of the line of sight is increased, a shake width of the line-of-sight center coordinates also becomes large. The aberration sensitivity measurement system 5 is configured to employ coordinate data in the section of 90% without employing coordinate data of 5% on each of the upper and lower sides in the coordinate axis direction. For this reason, with the aberration sensitivity measurement system 5, even in a case where the shake width of the line of sight is large, it is possible to increase the accuracy of the measurement by eliminating an abnormal coordinate value caused by noise, blink, or the like during the measurement.

The aberration sensitivity measurement system 5 sets a coordinate value (clip coordinates [-1,1]) of a central value of the employed valid coordinate data VD2 (that is, the coordinate data in the section of 90%) as an origin O of the aberration function.

That is, the aberration sensitivity measurement system 5 sets the center of the distribution of the valid coordinate data VD2L of the left eye as an aberration origin CL of the left eye and sets the center of the distribution of the valid coordinate data VD2R of the right eye as an aberration origin CR of the right eye.

That is, the aberration sensitivity measurement system 5 obtains the aberration origin CL of the left eye and the aberration origin CR of the right eye of the subject independently.

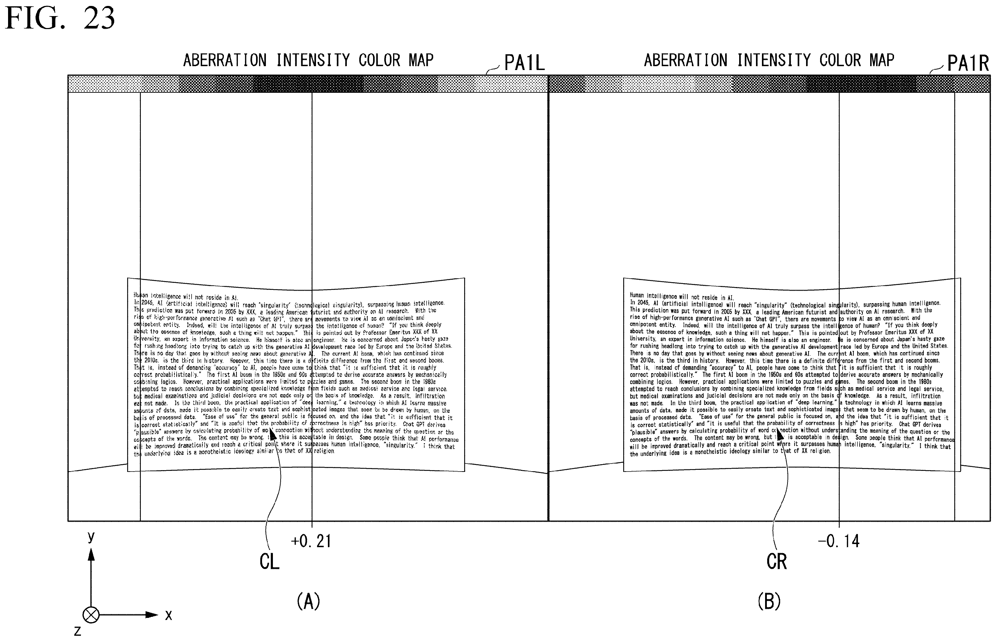

FIG. 23 is a diagram showing an example of blur aberration-applied images PA1 that are presented by the aberration sensitivity measurement system 5 of the present embodiment. The aberration sensitivity measurement system 5 presents images PA1 applied with blur aberration individually for the left eye and the right eye of the subject. In the drawing, an example of a blur aberration-applied image PA1L for a left eye and a blur aberration-applied image PA1R for a right eye is shown. In the blur aberration-applied images PA1, an aberration intensity is set such that an amount (blur amount) of a blur region B becomes greater than that near the aberration origin CL.

The aberration sensitivity measurement system 5 generates the image PA1L applied with blur aberration for the left eye, which is presented to the left eye of the subject, on the basis of the aberration origin CL of the left eye. The aberration sensitivity measurement system 5 generates the image PA1R applied with blur aberration for the right eye, which is presented to the right eye of the subject, on the basis of the aberration origin CL of the left eye.

Accordingly, with the aberration sensitivity measurement system 5, it is possible to match the center of the aberration distribution with the center of the line of sight according to the right and left eyes of the subject individual.

### [Aberration Condition]

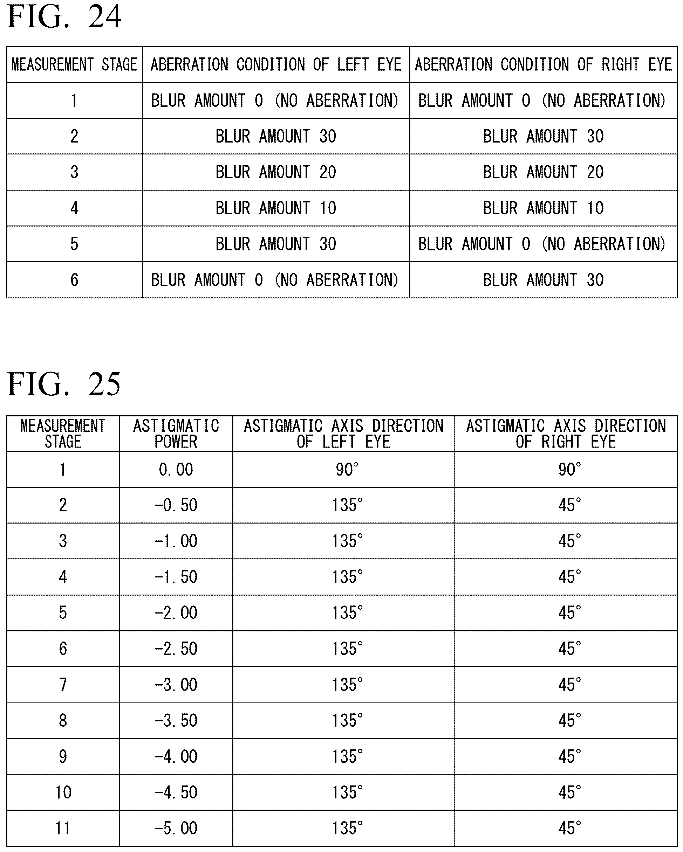

The aberration sensitivity measurement system 5 gets the subject to experience a scene while changing the aberration conditions of the right and left displays, and records data of the line of sight or the head posture and an appearance of a subjectivity level. A specific example of an aberration condition is shown in FIGS. 24 to 28.

### [Aberration Condition (1) Binocular Blur Sensitivity Test]

FIG. 24 is a diagram showing an example of an aberration condition for blur aberration of the present embodiment. As an example, the aberration sensitivity measurement system 5 performs a measurement of binocular blur sensitivity under conditions shown in the drawing. A measurement stage is an order of an aberration condition that is shown to the subject by the aberration sensitivity measurement system 5. The aberration sensitivity measurement system 5 may perform the measurement stages (that is, an order) randomly, not in an order shown in the drawing. Further, the aberration sensitivity measurement system 5 may repeatedly present the same measurement stage to the subject.

Like a measurement stage 5 or a measurement stage 6 shown in the drawing, the aberration sensitivity measurement system 5 of the present embodiment can present images individually applied with different aberrations to the left eye and the right eye of the subject.

### [Aberration Condition (2) Binocular Distortion Sensitivity Test]

FIG. 25 is a diagram showing an example of an aberration condition for distortion aberration of the present embodiment. As an example, the aberration sensitivity measurement system 5 performs a measurement of binocular distortion sensitivity under conditions shown in the drawing. The aberration sensitivity measurement system 5 may perform the measurement stages (that is, an order) randomly, not in an order shown in the drawing. Further, the aberration sensitivity measurement system 5 may repeatedly present the same measurement stage to the subject.

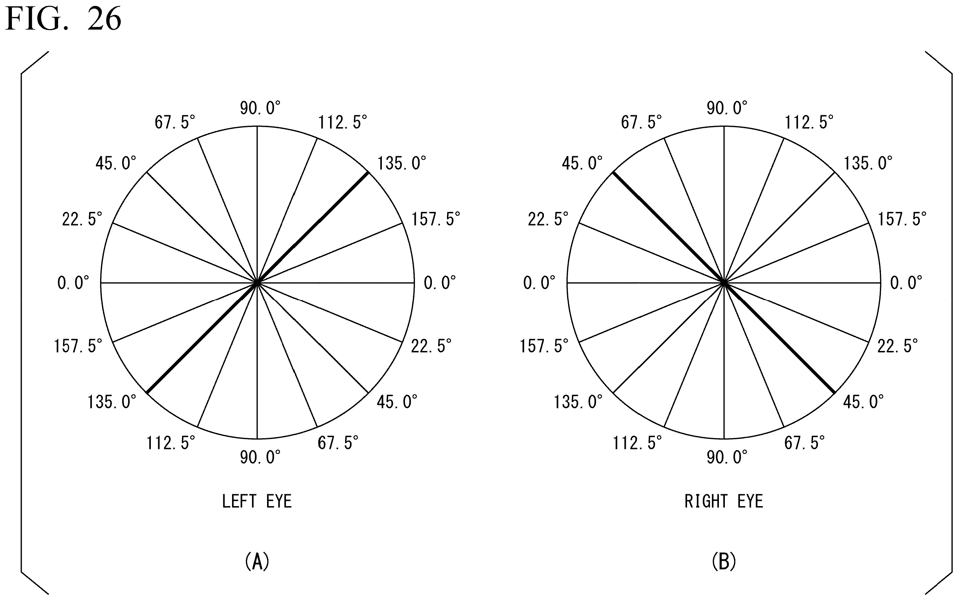

FIG. 26 is a diagram showing an example of conditions for an astigmatic axis for a left eye and an astigmatic axis for a right eye in a binocular distortion sensitivity test.

The aberration sensitivity measurement system 5 of the present embodiment can present images individually applied with different aberration conditions to the left eye and the right eye of the subject. Accordingly, the aberration sensitivity measurement system 5 can present images individually applied with different aberration conditions according to the astigmatic axis of the left eye and the astigmatic axis of the right eye of the subject, for example.

In the above-described example, an example of an aberration condition in a case where the astigmatic axis is fixed, and astigmatic power is changed is shown. As a modification example, astigmatic power may be fixed, and the astigmatic axis may be changed.

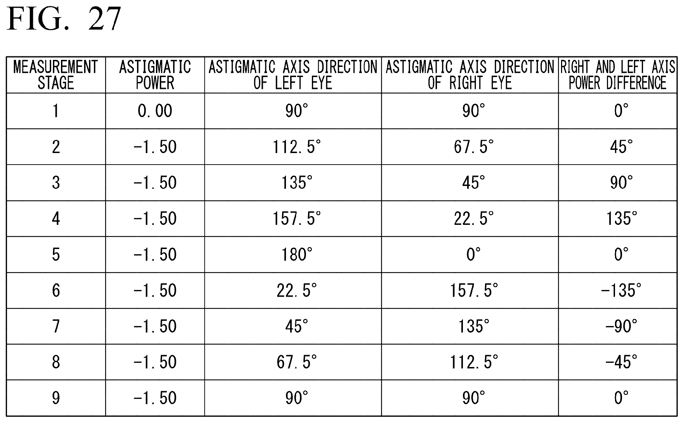

FIG. 27 is a diagram showing a modification example of an aberration condition for distortion aberration of the present embodiment.

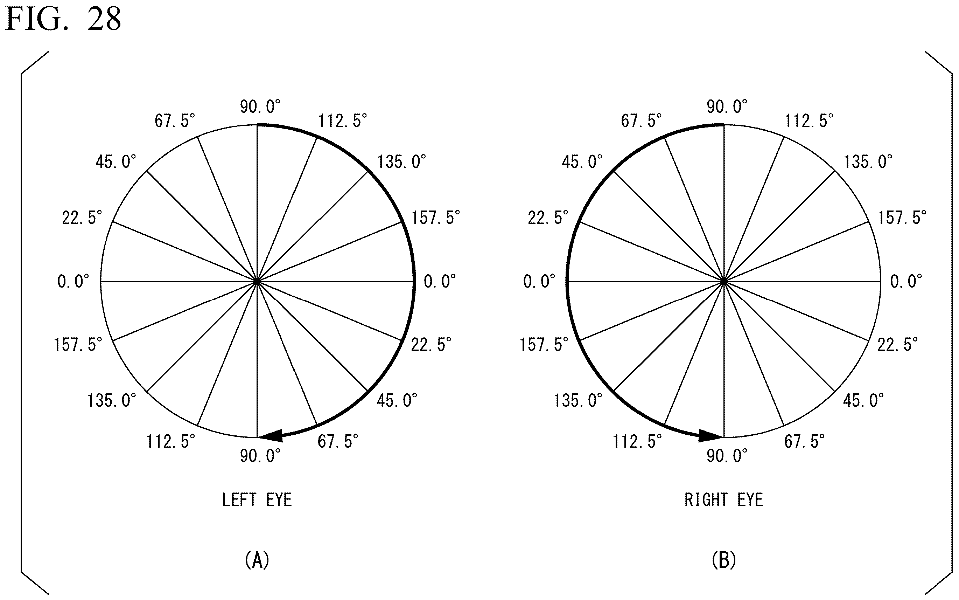

FIG. 28 is a diagram showing a modification example of conditions for an astigmatic axis of a left eye and an astigmatic axis of a right eye in a binocular distortion sensitivity test.

The aberration sensitivity measurement system 5 may perform a measurement by a combination of acquiring a condition for the astigmatic axis on which the subject feels the worst visual comfort, and then, changing astigmatic power with respect to the astigmatic axis.

Further, the aberration sensitivity measurement system 5 may perform a binocular sensitivity test while combining both blur aberration and distortion aberration.

### [Example of Measurement Result]

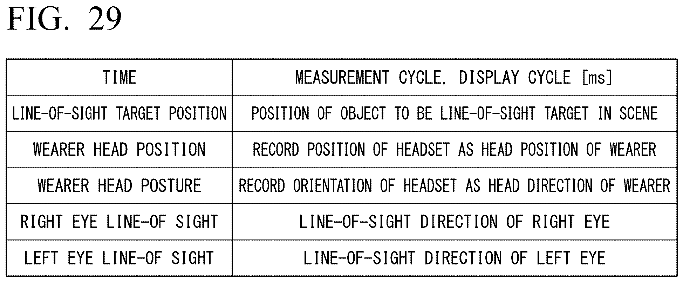

FIG. 29 is a diagram showing an example of a measurement result by the aberration sensitivity measurement system 5 of the present embodiment. In the measurement result, there are numerical data that is acquired from the display device 50 and data of a subjective visual comfort level that is acquired by asking to the subject.

### [Example of Analysis Result]

Next, an example of an analysis result by the aberration sensitivity measurement system 5 will be described.

### [Analysis Result (1) Change in Collision Position of Head Direction Extension Line and Virtual Object]

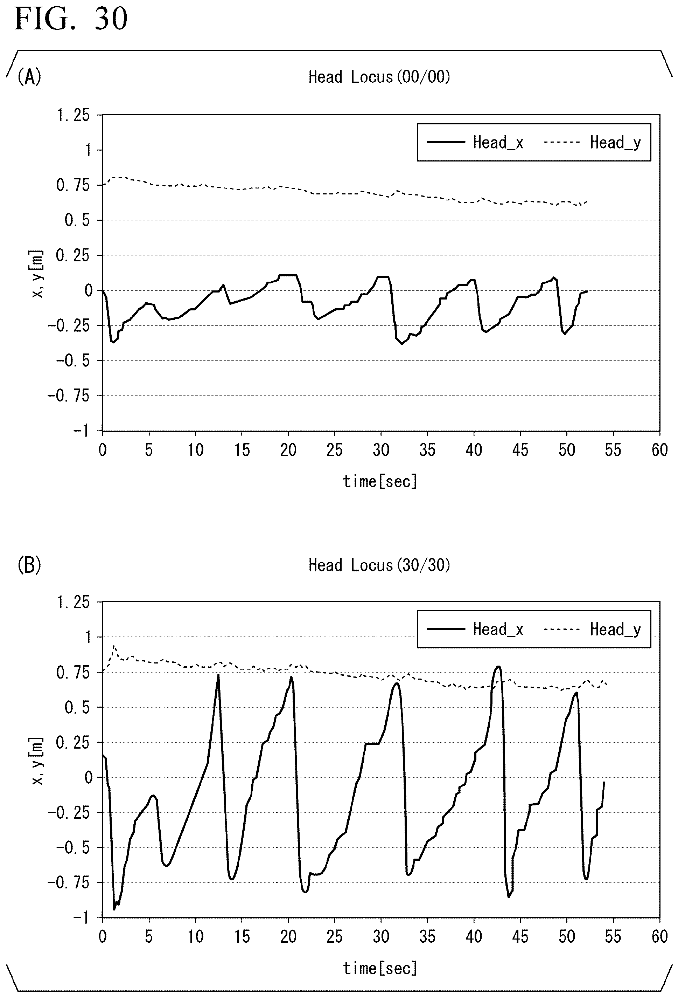

FIG. 30 is a diagram showing a first example of an analysis result by the aberration sensitivity measurement system 5 of the present embodiment. The aberration sensitivity measurement system 5 calculates an intersection of a virtual object presented in the virtual space VS and an extension line of a direction of the head of the subject in the virtual space VS. In (A) of the drawing, change with time in movement of the intersection in the x-axis direction and the y-axis direction in a state in which aberration is comparatively small is shown. In (B) of the drawing, change with time in movement of the intersection in the x-axis direction and the y-axis direction in a state in which aberration is comparatively large is shown.

It is analyzed that the subject in this example tends to significantly shake the head right and left when aberration increases. In this case, the aberration sensitivity measurement system 5 can analyze a relationship between the amount of aberration and the state of movement of the head.

### [Analysis Result (2) Change in Collision Position of Line-of-Sight Direction Extension Line and Virtual Object]

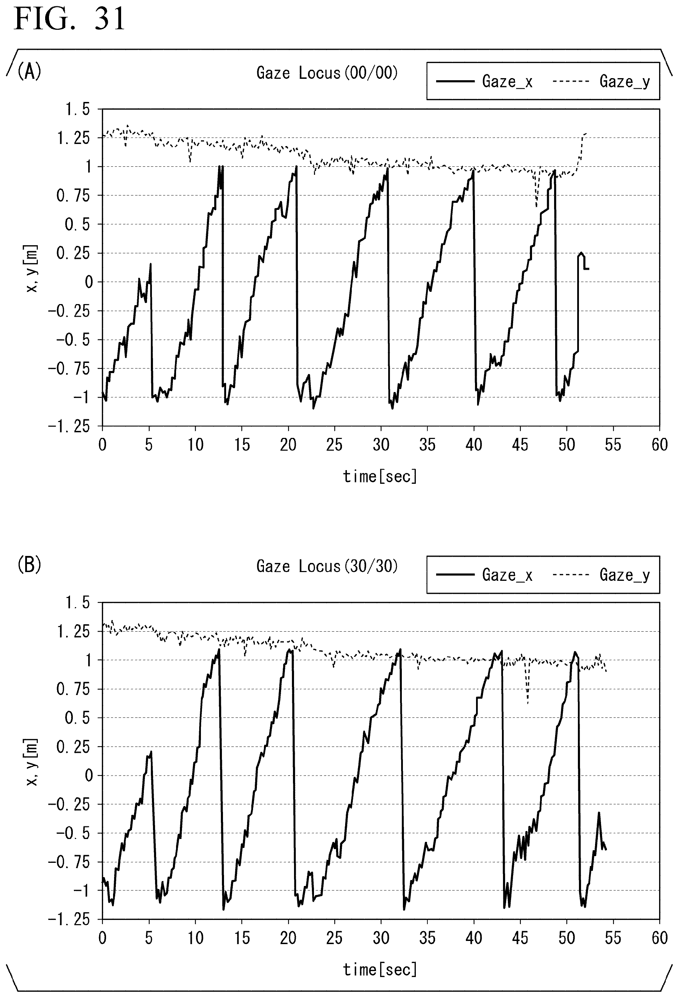

FIG. 31 is a diagram showing a second example of an analysis result by the aberration sensitivity measurement system 5 of the present embodiment. The aberration sensitivity measurement system 5 calculates an intersection of a virtual object presented in the virtual space VS and an extension line of a direction of the line of sight of the subject in the virtual space VS. In (A) of the drawing, change with time in movement of the intersection in the x-axis direction and the y-axis direction in a state in which aberration is comparatively small (for example, a state in which aberration is zero) is shown. In (B) of the drawing, change with time in movement of the intersection in the x-axis direction and the y-axis direction in a state in which aberration is comparatively large is shown.

A range in which the subject in this example views the virtual object is substantially the same regardless of the magnitude of aberration. From analogy based on the result of the above-described analysis result (1), it is analyzed that the subject tends to significantly shake right and left the line of sight when aberration is small, and tends to slightly shake the line of sight when aberration is large. In this way, the aberration sensitivity measurement system 5 can analyze a relationship between the amount of aberration and the state of movement of the line of sight (for example, whether the line of sight is surely tracing a target object).

### [Analysis Result (3) Line-of-Sight Distribution]

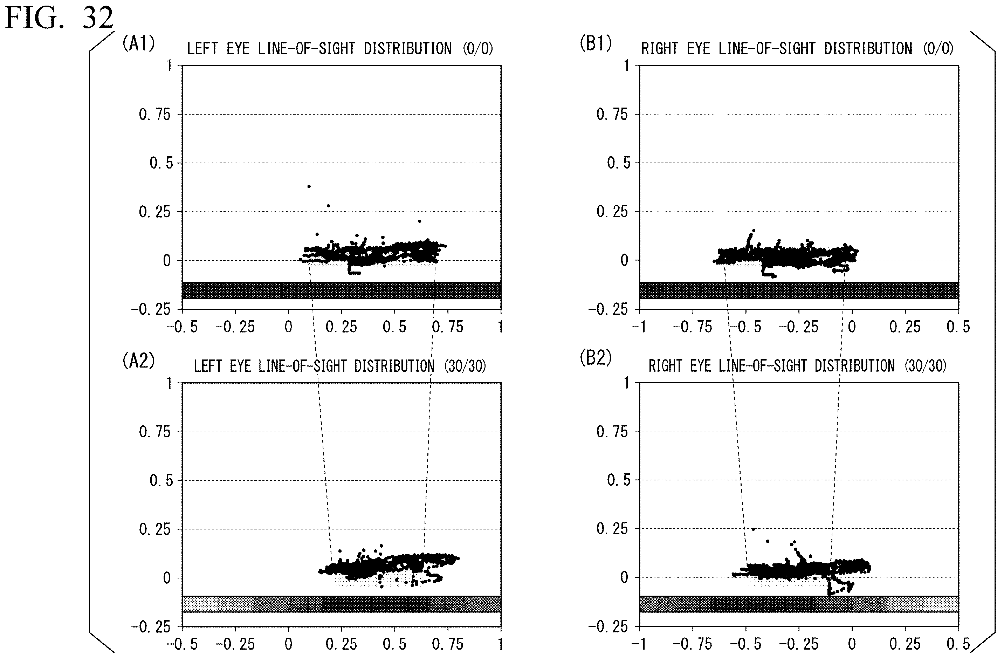

FIG. 32 is a diagram showing a third example of an analysis result by the aberration sensitivity measurement system 5 of the present embodiment. The aberration sensitivity measurement system 5 calculates a distribution (line-of-sight distribution) of an intersection between a plane before each of the right and left eyes in the virtual space VS and a line of sight of the subject.

(A1) of the drawing shows a line-of-sight distribution of the left eye of the subject in a state in which aberration is comparatively small (for example, a state in which aberration is zero). (B1) of the drawing shows a line-of-sight distribution of the right eye of the subject in a state in which aberration is comparatively small (for example, a state in which aberration is zero).

(A2) of the drawing shows a line-of-sight distribution of the left eye of the subject in a state in which aberration is comparatively large. (B2) of the drawing shows a line-of-sight distribution of the right eye of the subject in a state in which aberration is comparatively large.

For the line-of-sight distribution, a distribution range of 90% (a distribution range indicated by the above-described valid coordinate data VD2) is used.

The aberration sensitivity measurement system 5 analyzes how much the subject is moving the eye right and left and up and down. In an example shown in the drawing, to allow the line of sight of the subject to avoid a portion in which aberration is large, an example where a width of the distribution range of 90% is narrowed is shown.

### [Analysis Result (4) Head Rotation Angle Range]

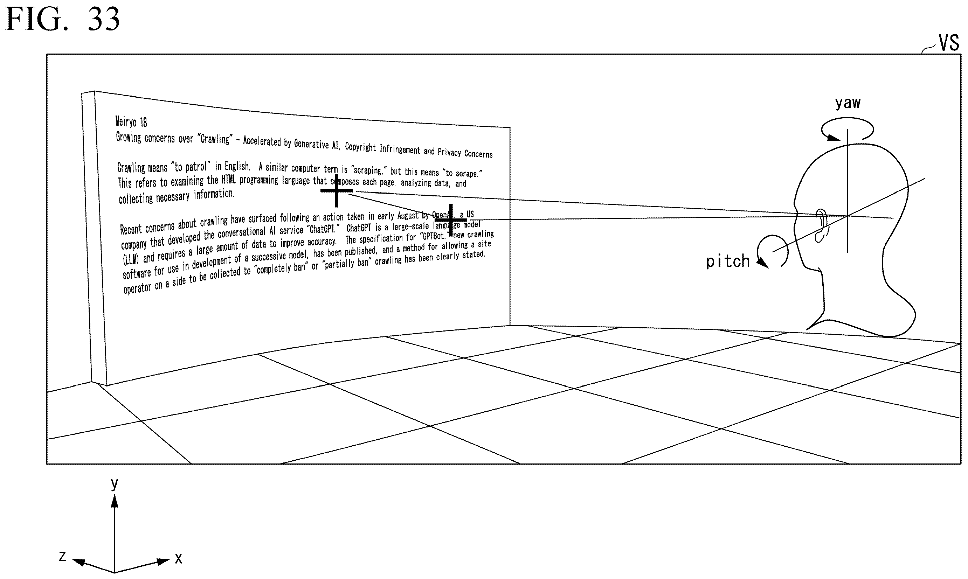

FIG. 33 is a diagram showing a fourth example of an analysis result by the aberration sensitivity measurement system 5 of the present embodiment. The aberration sensitivity measurement system 5 calculates a section width of 90% in an angle distribution when a distribution of a collision position of a head direction extension line and an object (display) is converted into a distribution of an angle.

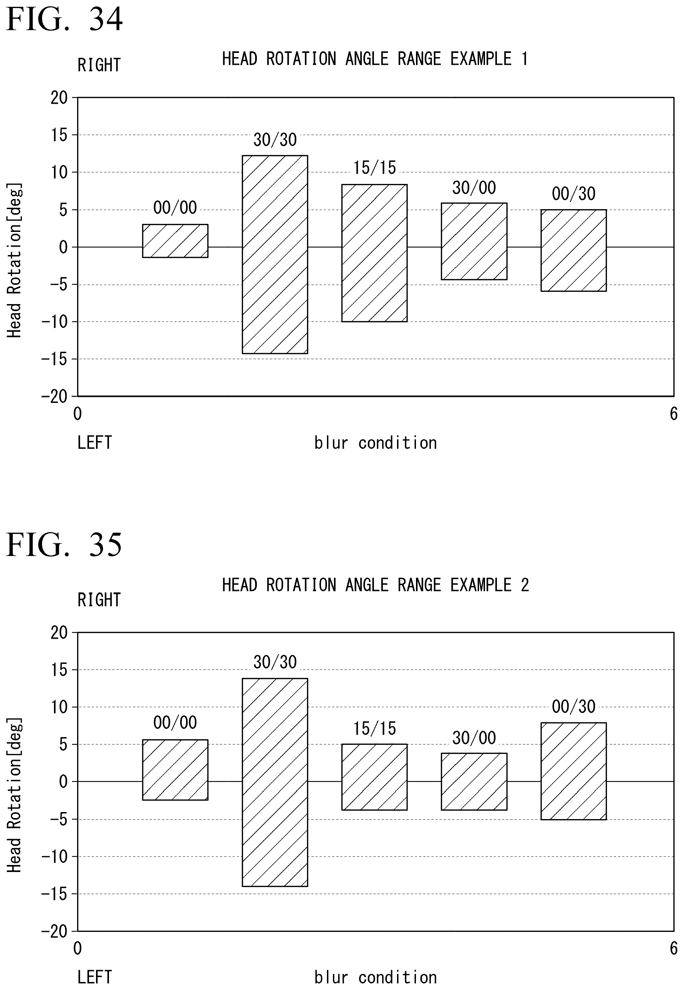

FIG. 34 is a diagram showing a first example of a calculation result of an angle distribution of a head rotation angle.

FIG. 35 is a diagram showing a second example of a calculation result of an angle distribution of a head rotation angle.

The aberration sensitivity measurement system 5 calculates an Euler angle of the head of the subject on the basis of a reference direction (for example, a front direction) of the head and a current direction of the head of the subject. In an example shown in the drawing, the aberration sensitivity measurement system 5 calculates rotation angles of a yaw component and a pitch component of an Euler angle.

The aberration sensitivity measurement system 5 presents a maximum value and a minimum value of the calculated rotation angles in a graph format for each aberration condition.

### [Analysis Result (5) Right/Left Eye Rotation Angle]

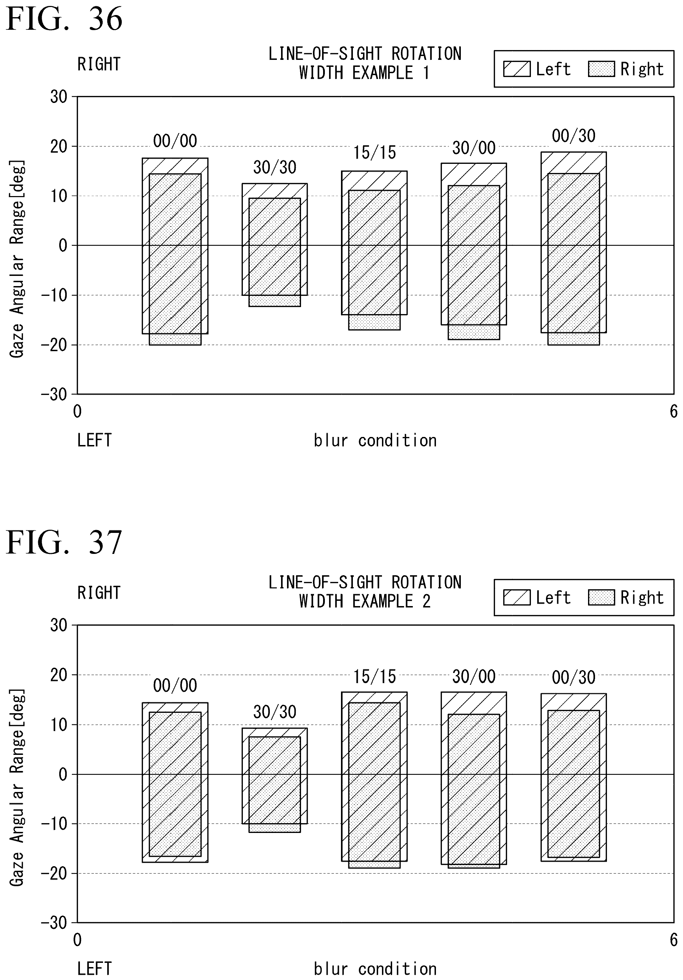

FIG. 36 is a diagram showing a first example of a calculation result of an angle distribution of a right/left eye rotation angle.

FIG. 37 is a diagram showing a second example of a calculation result of an angle distribution of a right/left eye rotation angle.

The aberration sensitivity measurement system 5 calculates rotation angles in a lateral direction (yaw component) and a longitudinal direction (pitch component) of the line-of-sight distribution of each of the left eye and the right eye. The aberration sensitivity measurement system 5 presents a maximum value and a minimum value of the calculated rotation angles in a graph format for each aberration condition.

### [Analysis Result (6) Binocular Line-of-Sight Rotation Width]

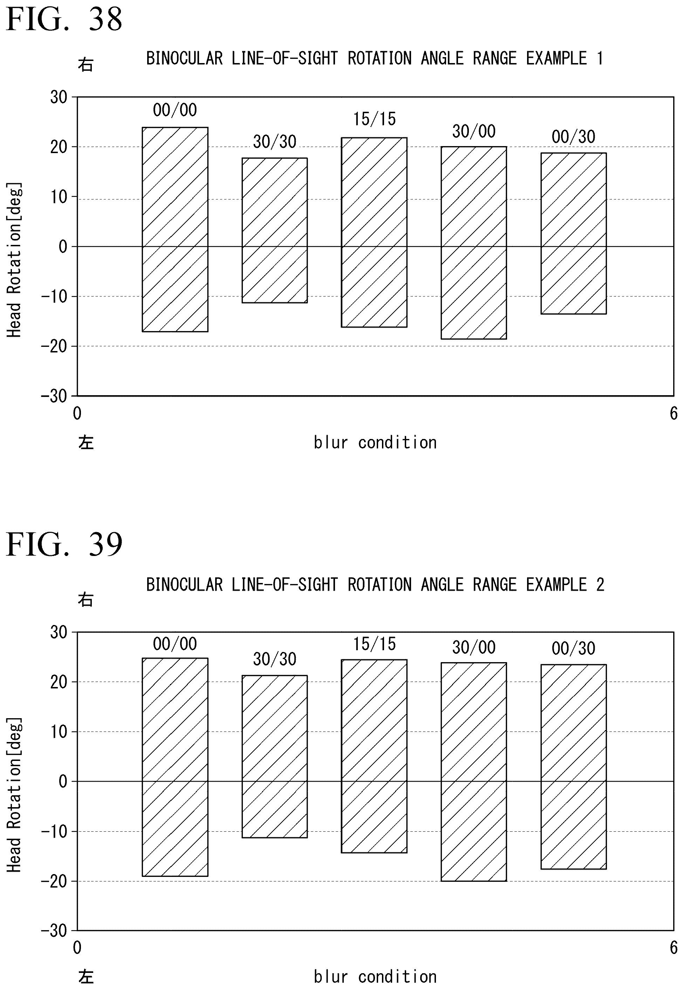

FIG. 38 is a diagram showing a first example of a calculation result of a binocular line-of-sight rotation width.

FIG. 39 is a diagram showing a second example of a calculation result of a binocular line-of-sight rotation width.

Here, the binocular rotation width is a width of a range in which a line-of-sight rotation width of the left eye and a line-of-sight rotation width of the right eye overlap each other. The aberration sensitivity measurement system 5 analyzes start and end values of the rotation width, a size, and an influence of aberration. For example, the aberration sensitivity measurement system 5 calculates the influence of aberration by subtracting a result with no aberration from a result with aberration. As an influence value is smaller, there is less influence of aberration. For this reason, it is possible to extract an aberration condition that influenced by a threshold value and to use the extracted aberration condition as an acceptable aberration level of design.

### [Analysis Result (7) Average Aberration Addition Level]

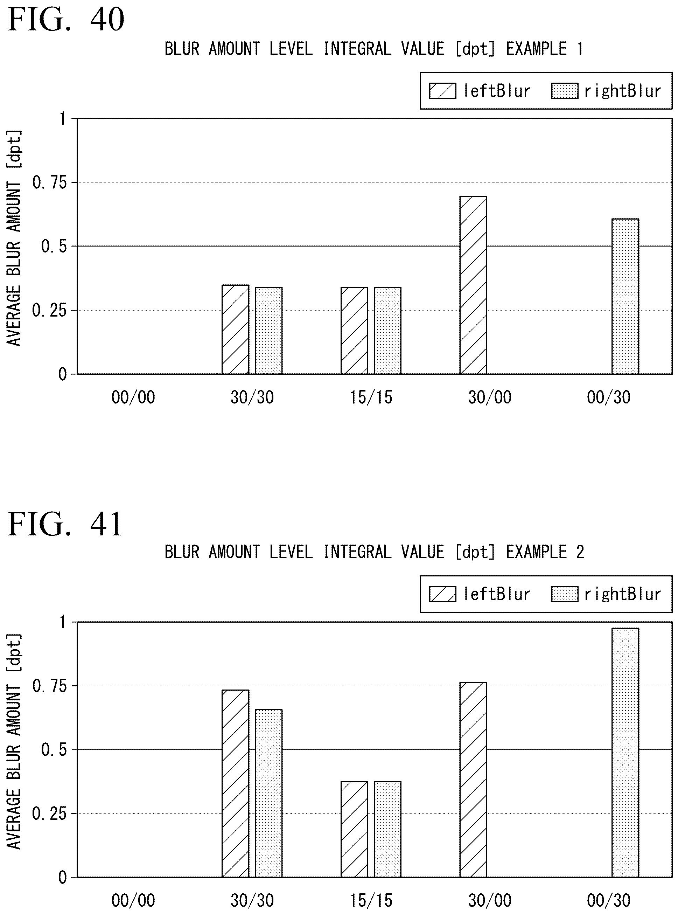

FIG. 40 is a diagram showing a first example of a calculation result of an average aberration addition level.

FIG. 41 is a diagram showing a second example of a calculation result of an average aberration addition level.

The average aberration addition level indicates an aberration amount portion that is used when the line of sight is viewing in the visual field. The aberration sensitivity measurement system 5 calculates an aberration amount of a line-of-sight position for each sample time. The aberration sensitivity measurement system 5 integrates a calculated aberration amount graph and sets a value obtained by dividing an integral value by a time as an average aberration addition level. In a case where both eyes have the same aberration, the average aberration addition level can be used as a sensitivity visual target of the subject to blur.

### [Analysis Result (8) Average Aberration Addition Level]

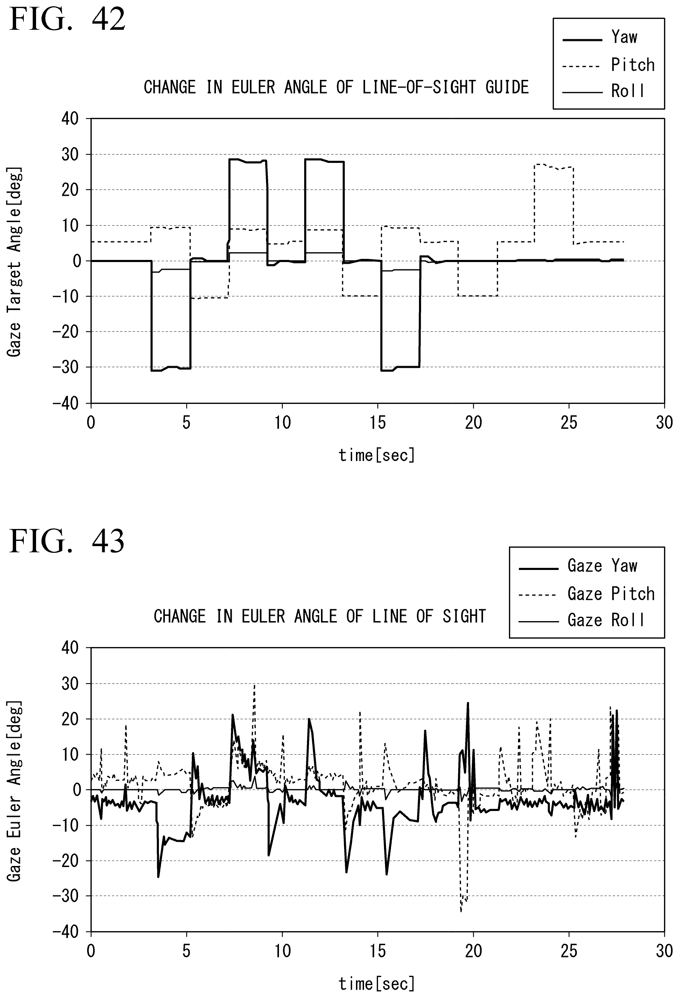

FIG. 42 is a diagram showing an example of an Euler angle in the virtual space VS.

FIG. 43 is a diagram showing an example of change in line-of-sight angle in gazing steadily at a line-of-sight guide object.

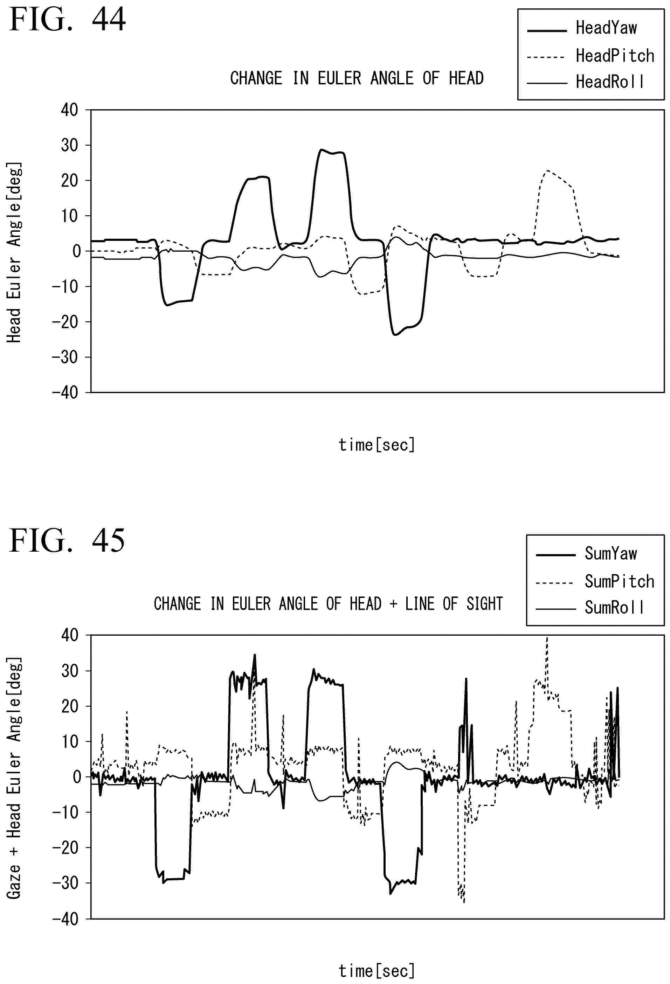

FIG. 44 is a diagram showing an example of change in head angle in gazing steadily at a line-of-sight guide object.

FIG. 45 is a diagram showing an example of a result of composition of change in line-of-sight angle and change in head angle.

In a case where a visual target (line-of-sight guide object) is displayed and moved in the measurement scene, the aberration sensitivity measurement system 5 analyzes a shift amount between the visual target and the line of sight of the subject on the time axis. The magnitude of the shift amount is proportional to the magnitude of sensitivity. The shift amount is represented as a distance between the visual target and the line-of-sight intersection in a plane perpendicular to the line of sight or a difference in Euler rotation (around the yaw axis, around the pitch axis, and around the roll axis) of the line of sight.

The aberration sensitivity measurement system 5 sets a value obtained by integrating a difference between an Euler angle of the line-of-sight guide and an Euler angle at which the shift amount of the visual target and the line of sight match each other, on the time axis.

### [Analysis Result (9) Average Pupil Diameter]

The aberration sensitivity measurement system 5 may calculate a pupil diameter. Specifically, the aberration sensitivity measurement system 5 rearranges measurement results of the pupil diameter in an ascending order or a descending order and employs a predetermined range (for example, a range of 90%) in a center portion of the arrangement order as valid data. The aberration sensitivity measurement system 5 sets an intermediate value (for example, a median value or an average value) of the employed valid data as an average pupil diameter.

### [Analysis Result (10) Number of Blinks]

The aberration sensitivity measurement system 5 may calculate the number of blinks. Specifically, the aberration sensitivity measurement system 5 counts the number of blinks by determining the presence or absence of a blink on the basis of change with time of an opening degree of the eye. The aberration sensitivity measurement system 5 evaluates the number of blinks per unit time by dividing the counted number of blinks by a measurement time.

### [Usage in Design Visual Target of Spectacle Lens]

The aberration sensitivity measurement system 5 outputs the above-described analysis result as a design visual target of the spectacle lens reflecting an individual difference of the subject or a difference in sensitivity of the right and left eyes.

### [Usage in Design Visual Target of Spectacle Lens (1) Right and Left Sensitivity Balance]

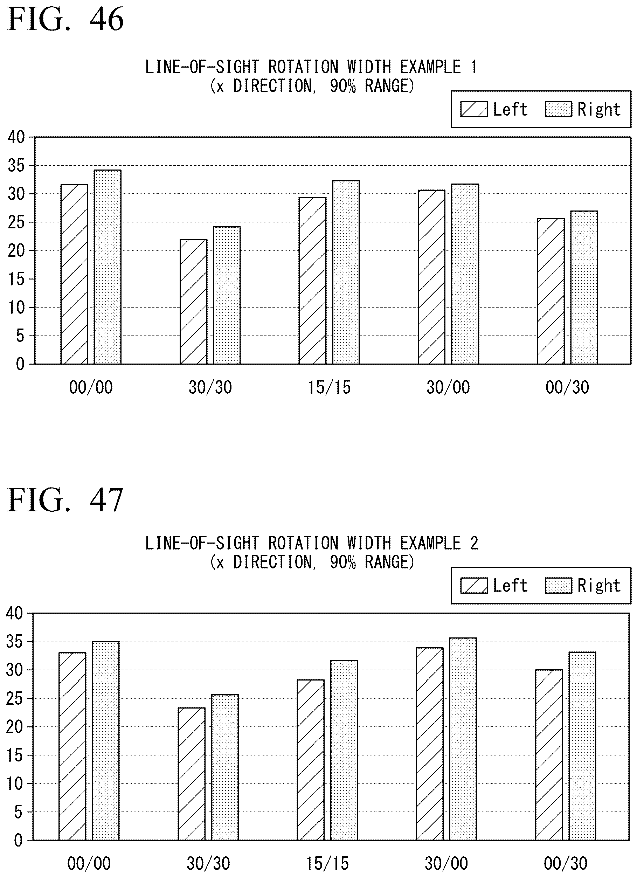

FIG. 46 is a diagram showing a first example of an analysis result of a right and left sensitivity balance.

FIG. 47 is a diagram showing a second example of an analysis result of a right and left sensitivity balance.

The aberration sensitivity measurement system 5 outputs a result of comparison of the rotation angles of the right eye and the left eye for each aberration condition as the design visual target of the spectacle lens reflecting the individual difference of the subject or the difference in sensitivity of the right and left eyes.

### [Usage in Design Visual Target of Spectacle Lens (2) Acceptable Blur Amount in :ine-of-Sight Movement]

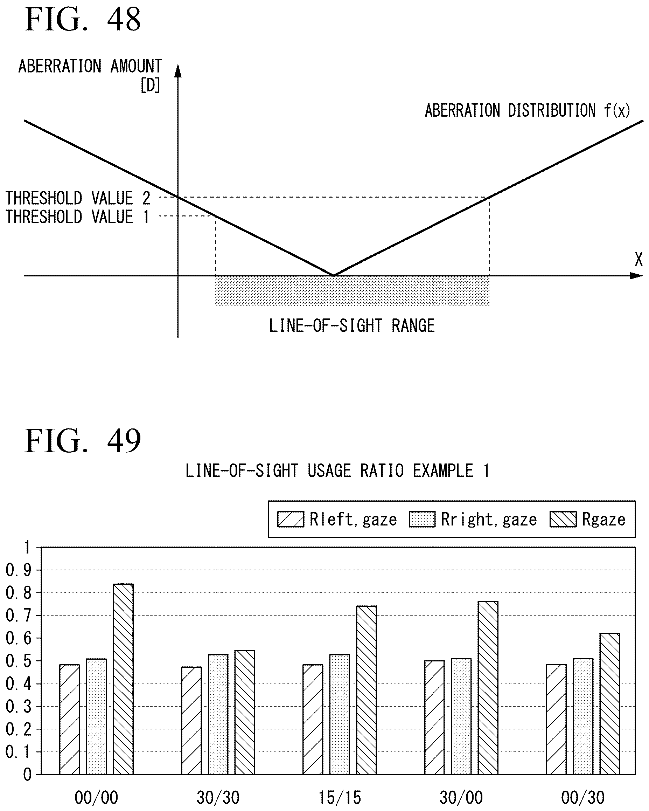

FIG. 48 is a diagram showing an example of a correspondence relationship between an aberration amount and a line-of-sight range of the subject. The aberration sensitivity measurement system 5 detects a condition on which the rotation width significantly falls (that is, the subject shakes the head while avoiding aberration and views). The aberration sensitivity measurement system 5 calculates to which portion the subject is rotating the eye in the aberration distribution of the condition.

A determination threshold value of the line-of-sight range can be set to an aberration amount of one (for example, a smaller one) of values of a start point and an end position of the line-of-sight range.

### [Usage in design visual target of spectacle lens (3) line-of-sight usage ratio]

A proportion of the line of sight when the subject rotates the head and the line of sight to view things is defined as a line-of-sight usage ratio. The aberration sensitivity measurement system 5 calculates change in ratio of a case where the condition of aberration is changed compared to a case where both eyes can view clearly (00/00).

A calculation expression of the line-of-sight usage ratio is shown in Expression (16).
[Equation 16] ${R}_{gaze} = \frac{{G}_{both}}{H + {G}_{both}}$ (16)

A calculation expression of a right/left line-of-sight usage ratio is shown in Expression (17).
[Equation 17] $\left[\begin{matrix}{R}_{left , gaze} = \frac{{G}_{left}}{{G}_{left} + {G}_{right}} \\ {R}_{right , gaze} = \frac{{G}_{right}}{{G}_{left} + {G}_{right}} = 1 - {R}_{left , gaze}\end{matrix}\right.$ (17)

FIG. 49 is a diagram showing a first example of an analysis result of a line-of-sight usage ratio.

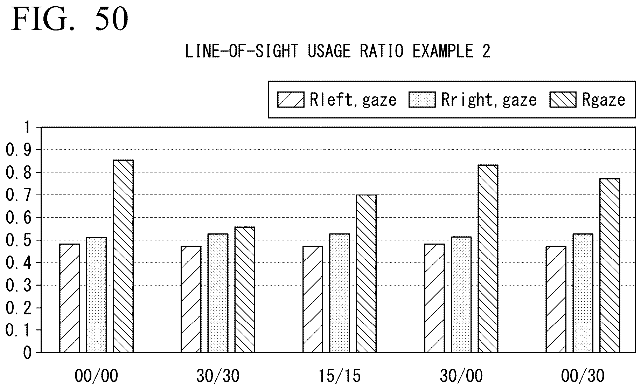

FIG. 50 is a diagram showing a second example of an analysis result of a line-of-sight usage ratio.

The aberration sensitivity measurement system 5 outputs a result of calculation of the line-of-sight usage ratio for each aberration condition as a design index of the spectacle lens reflecting the individual difference of the subject or the difference in sensitivity of the right and left eyes.

### [Usage in design visual target of spectacle lens (4) acceptable distortion amount]

The aberration sensitivity measurement system 5 outputs astigmatic power based on a subjective evaluation result (for example, level 3: slightly feels discomfort) of a distortion sensitivity test as acceptable distortion power and as a design index of the spectacle lens reflecting the individual difference of the subject or the difference in sensitivity of the right and left eyes.

### [Usage in Design Visual Target of Spectacle Lens (5) Acceptable Astigmatic Axis Right/Left Difference]

The aberration sensitivity measurement system 5 outputs astigmatic power based on a subjective evaluation result (for example, level 3: slightly feels discomfort) in a case where the difference of the right and left astigmatic axes is changed, as a right/left acceptable astigmatic axis power difference and as a design index of the spectacle lens reflecting the individual difference of the subject or the difference in sensitivity of the right and left eyes.

As described above, with the aberration sensitivity measurement system 5, it is possible to acquire data (for example, aberration sensitivity) to be a design index of a spectacle lens reflecting an individual difference of the subject or a difference in sensitivity between the right and left eyes.

Further, with the aberration sensitivity measurement system 5, to acquire aberration sensitivity with a virtual spectacle lens through image processing, for example, it is possible to measure a sensitivity parameter while allowing the subject to experience an appearance with aberration that is difficult to realize with a physical test lens.

### [Spectacle Lens Ordering and Order Reception System]

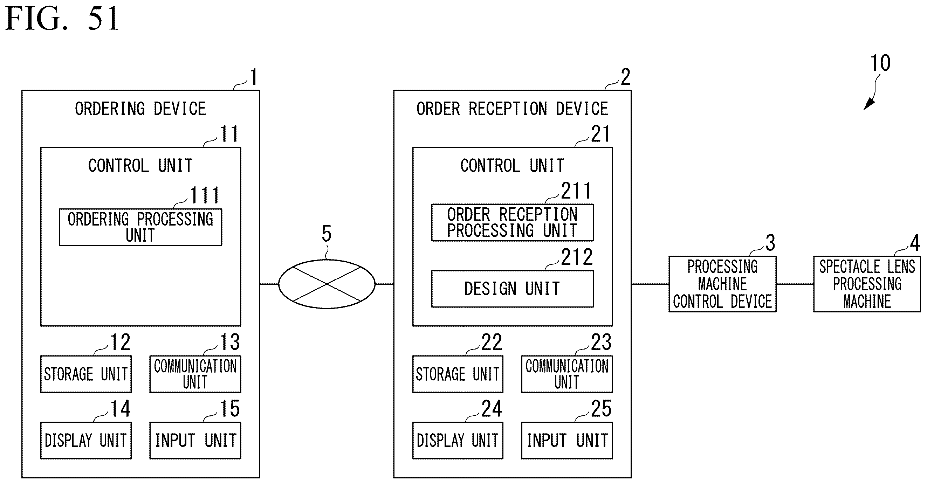

FIG. 51 is a diagram showing an example of a functional configuration of a spectacle lens ordering and order reception system 10 of the present embodiment. The spectacle lens ordering and order reception system 10 includes an ordering device 1 that is provided in an optician's store (that is, an ordering party of a spectacle lens), an order reception device 2 that is provided in a lens manufacturer, a processing machine control device 3, and a spectacle lens processing machine 4.

The ordering device 1 and the order reception device 2 are connected in a communicative manner, for example, via a network 5 such as the Internet. Further, the processing machine control device 3 is connected to the order reception device 2, and the spectacle lens processing machine 4 is connected to the processing machine control device 3. In FIG. 7, for convenience of illustration, only one ordering device 1 is described, but actually a plurality of ordering devices 1 that are provided in a plurality of optician's stores are connected to the order reception device 2.

The ordering device 1 is a computer that performs ordering processing of a spectacle lens, and includes a control unit 11, a storage unit 12, a communication unit 13, a display unit 14, and an input unit 15. The control unit 11 includes a processor such as a CPU, and controls the ordering device 1 by executing a program stored in the storage unit 12. The control unit 11 includes an ordering processing unit 111 that performs ordering processing of a spectacle lens. The communication unit 13 performs communication with the order reception device 2 via the network 5. The display unit 14 is a display device such as a liquid crystal display or a CRT, and displays an ordering screen for inputting information (ordering information) of a spectacle lens to order, or the like. The input unit 15 includes an input device such as a mouse or a keyboard. The ordering information according to the content of the ordering screen is input via the input unit 15.

The display unit 14 and the input unit 15 may be configured integrally by a touch panel or the like.

The order reception device 2 is a computer that performs order reception processing or design processing of a spectacle lens, arithmetic operation processing of optical performance, or the like, and includes a control unit 21, a storage unit 22, a communication unit 23, a display unit 24, and an input unit 25. The control unit 21 includes a processor such as a CPU, and controls the order reception device 2 by executing a program stored in the storage unit 22. The control unit 21 includes an order reception processing unit 211 that performs order reception processing of a spectacle lens, and a design unit 212 that performs design processing of the spectacle lens. The communication unit 23 performs communication with the ordering device 1 via the network 5 or performs communication with the processing machine control device 3. The storage unit 22 stores various kinds of data for designing a spectacle lens in a readable manner. The display unit 24 is a display device such as a liquid crystal display or a CRT, and displays a design result of a spectacle lens, or the like. The input unit 25 includes an input device such as a mouse or a keyboard.

The display unit 24 and the input unit 25 may be configured integrally by a touch panel or the like. Further, the functions of the order reception device 2 may be performed by the order reception device including the order reception processing unit 211 and a design device including the design unit 212.

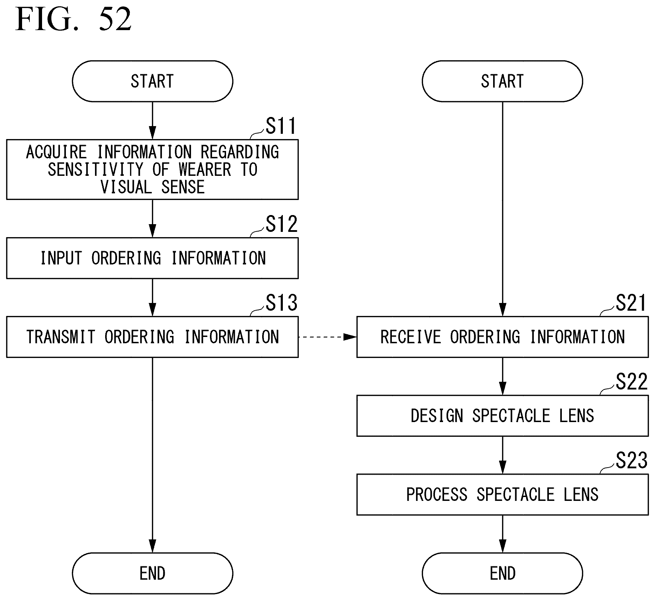

FIG. 52 is a diagram illustrating an example of a flow of an operation of the spectacle lens ordering and order reception system of the present embodiment. In the spectacle lens ordering and order reception system 10, a procedure for providing a spectacle lens will be described using a flowchart shown in the drawing. A procedure S11 to S13 that is performed on an optician's store side is shown on the left side of the drawing, and a procedure S21 to S23 that is performed on a lens manufacturer side is shown on the right side of the drawing. In a manufacturing method of a spectacle lens in the spectacle lens ordering and order reception system 10, a spectacle lens is designed by the above-described design method of the spectacle lens.

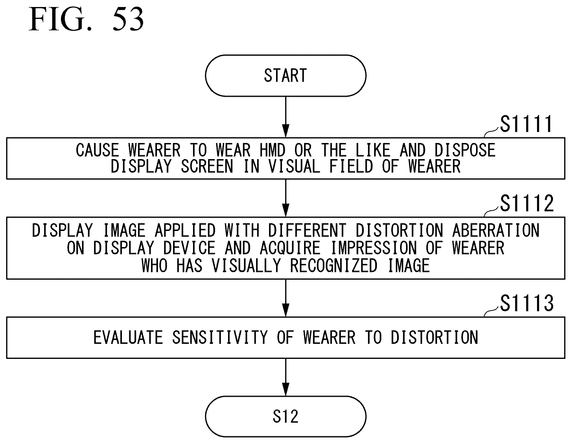

In Step S11, the ordering party acquires information regarding sensitivity of the subject to a visual sense. In the present embodiment, as shown in FIG. 53, the ordering party performs an aberration sensitivity examination with the subject as a subject and acquires information regarding sensitivity of the subject to distortion.

FIG. 53 is a diagram illustrating an example of a flow of a detailed operation in Step S11. In Step S1111, the ordering party causes the subject to wear the display device 50 such as the HMD and disposes the display screen 501 in the visual field of the subject. After Step S1111 ends, the process proceeds to Step S1112.

In Step S1112, the ordering party sequentially displays a plurality of distortion aberration-applied images Y including different kinds of distortion on the display screen 501 to cause the subject to visually recognize the distortion aberration-applied image Y and acquires an impression of the subject who has visually recognized the distortion aberration-applied image Y. The ordering party displays the distortion aberration-applied image Y in the regions of the display screen 501 corresponding to the lateral portions La1 and La2 of the progressive refractive power lens as shown in FIG. 6, for example. The ordering party fixes a distortion direction of the distortion aberration-applied image Y to be displayed, increases a degree of distortion, and specifies the distortion aberration-applied image Y for which the subject replies that the distortion aberration-applied image Y is unacceptable. In addition, the ordering party fixes a distortion direction in a state in which the distortion direction is changed, increases a degree of distortion, and specifies the distortion aberration-applied image Y for which the subject replies that the distortion aberration-applied image Y is unacceptable. In this way, a plurality of distortion aberration-applied images Y having different degrees of distortion for one or a plurality of distortion directions are displayed. After Step S1112 ends, the process proceeds to Step S1113.

An order in which distortion aberrations having different degrees of distortion are applied is not particularly limited.

In Step S1113, the ordering party evaluates sensitivity to distortion in the visual field of the subject who has visually recognized the distortion aberration-applied image Y. The ordering party converts sensitivity of the subject to distortion into a numerical value on the basis of the reply from the subject who has visually recognized the distortion aberration-applied image Y, obtained in Step S1112 according to a criterion determined in advance and records the numerical value. For example, as described above, in the case of increasing the degree of distortion of the distortion aberration-applied image Y until the subject replies that the distortion aberration-applied image Y is unacceptable, a degree of distortion of the distortion aberration-applied image Y for which the subject initially replies that the distortion aberration-applied image Y is unacceptable or a degree of distortion of the distortion aberration-applied image Y that is a limit acceptable to the subject is acquired as a parameter (hereinafter, called a sensitivity parameter) indicating sensitivity of the subject to distortion. That is, the sensitivity parameter is determined on the basis of the image applied with the above-described specified distortion aberration. After Step S1113 ends, the process proceeds to Step S12.

Returning to FIG. 52, in Step S12, the ordering party determines ordering information of the spectacle lens including sensitivity to distortion in the visual field of the subject such as the sensitivity parameter acquired in Step S1113. Then, the ordering party causes the display unit 14 of the ordering device 1 to display an ordering screen and inputs the ordering information via the input unit 15.

In Step S13, the ordering device 1 transmits the ordering information to the order reception device 2 via the communication unit 13.

In Step S21, when the ordering information is received from the ordering device 1 via the communication unit 23, the order reception processing unit 211 of the order reception device 2 proceeds to Step S22. In Step S22, the design unit 212 of the order reception device 2 designs the spectacle lens on the basis of the received ordering information.

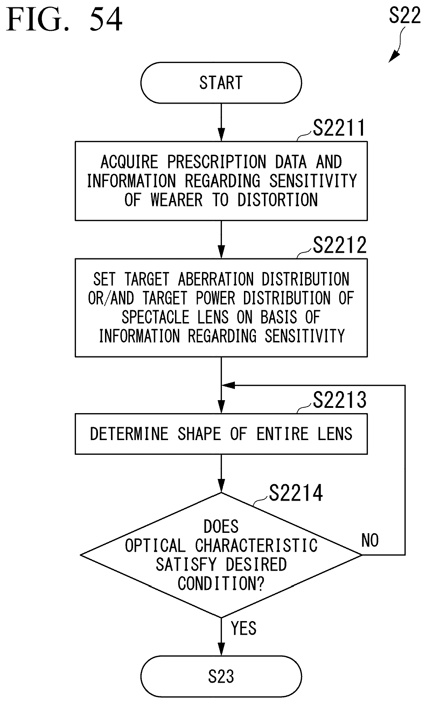

FIG. 54 is a diagram illustrating an example of a flow of a detailed operation in Step S22. In Step S2211, the order reception device 2 acquires prescription data of the spectacle lens, information regarding sensitivity of the subject to distortion, and/or a design parameter such as an index indicating a range in which astigmatism is small. The order reception device 2 also suitably acquires fitting parameters such as a forward tilt angle of a frame, a face form angle, and a cornea vertex distance. After Step S2211 ends, the process proceeds to Step S2212.

In Step S2212, the design unit 212 of the order reception device 2 sets a target aberration distribution or/and a target power distribution of the spectacle lens on the basis of information regarding sensitivity of the subject to distortion and/or the design parameter acquired in Step S2211.

In Step S2213, the order reception device 2 determines a shape of the entire lens of the spectacle lens on the basis of the set target aberration distribution or/and target power distribution. After Step S2213 ends, the process proceeds to Step S2214.

In Step S2214, the order reception device 2 determines whether an optical characteristic such as refractive power or astigmatism of the spectacle lens satisfies a desired condition. The desired condition refers to a condition that the entire prescription is satisfied while reflecting the sensitivity of the subject. In a case where the desired condition is satisfied, affirmative determination is made in Step S2214, the design processing ends, and the process proceeds to Step S23. In a case where the desired condition is not satisfied, negative determination is made in Step S2214, and the process returns to Step S2213.

Returning to FIG. 52, in Step S23, the order reception device 2 outputs design data of the spectacle lens designed in Step S22 to the processing machine control device 3. The processing machine control device 3 sends a processing instruction to the spectacle lens processing machine 4 on the basis of the design data output from the order reception device 2. As a result, the spectacle lens based on the design data is processed by the spectacle lens processing machine 4 and manufactured. The spectacle lens manufactured by the spectacle lens processing machine 4 is shipped to the optician's store, is fitted into a spectacle frame, and is provided to a customer (the subject or the wearer of the spectacles).

In the order reception device 2, the processing of receiving the ordering information from the ordering device 1, the processing of designing the spectacle lens on the basis of the received ordering information, and the processing of outputting the design data of the spectacle lens to the processing machine control device 3 are performed by the control unit 21 of the order reception device 2 executing a predetermined program installed in advance in the storage unit 22.

With the spectacle lens ordering and order reception system of the present embodiment, it is possible to design a spectacle lens matching the wearer on the basis of the sensitivity of each of both eyes of the subject (wearer) measured by the aberration sensitivity measurement system 5.

While the embodiment of the present invention has been described above in detail with reference to the drawings, a specific configuration is not limited to the embodiment, and various modifications may be made without departing from the spirit of the present invention. The configurations described in the respective embodiments described above may be combined.

The respective units provided in the respective devices in the above-described embodiment may be realized by dedicated hardware, or further, may be realized by a memory and a microprocessor.

The respective units provided in each device may be configured with a memory and a central processing unit (CPU), and the functions thereof may be realized by loading a program for realizing the functions of the respective units provided in the respective devices on a memory and executing the program.

Processing by the respective units provided in the control unit may be executed by recording the program for realizing the functions of the respective units provided in the respective devices on a computer-readable recording medium, and causing a computer system to read and execute the program stored in the recording medium. Moreover, "the computer system" stated herein includes an OS or hardware such as peripheral equipment.

"The computer system" also includes a homepage providing environment (or a display environment) when a WWW system is used.

"The computer-readable recording medium" refers to a portable medium such as a flexible disk, a magneto-optical disk, a ROM, or a CD-ROM, or a storage device such as a hard disk embedded in the computer system. In addition, "the computer-readable recording medium" includes a medium that dynamically holds a program for a short time like a communication line in a case where a program is transmitted via a network such as the Internet or a communication line such as a telephone line, or a medium that holds a program for a given time like a volatile memory in a computer system to be a server or a client in this case. Further, the above-described program may realize some of the above-described functions , and further, may realize the above-described functions in combination with the program already recorded in the computer system.

### REFERENCE SIGNS LIST

1 Ordering device
2 Order reception device
3 Processing machine control device
4 Spectacle lens processing machine
5 Aberration sensitivity measurement system
10 Terminal device
110 Arithmetic unit
111 Line-of-sight center position calculation unit
112 Aberration sensitivity information generation unit
113 Display control unit
120 Communication unit
121 Line-of-sight information acquisition unit
122 Head position and posture information acquisition unit
123 Image output unit
1231 First image output unit
1232 Second image output unit
130 Storage unit
140 Input unit
50 Display device
52 Support unit
56 Image forming lens
500 Display unit
501 Display screen
502 Angle detection unit
505 Communication unit
506 Line-of-sight detection unit
510 Image display control unit
VS Virtual space
VS61 Operation panel portion
VS62 Forward visual field portion
PG Guide object
PA1 Image applied with blur aberration
PA2 Image applied with distortion aberration

## Claims

1. A terminal device comprising:
a first image output unit configured to output image information of an image for a left eye, in which a first aberration distribution as an aberration distribution for a left eye is settable, to a left eye display unit configured to present an image to a left eye of a subject;
a second image output unit configured to output image information of an image for a right eye, in which a second aberration distribution as an aberration distribution for a right eye is settable, to a right eye display unit configured to present an image to a right eye of the subject;
a line-of-sight information acquisition unit configured to acquire line-of-sight information obtained by detecting movement of a line of sight of the subject presented with at least one of the image for the left eye and the image for the right eye; and
an aberration sensitivity information generation unit configured to generate aberration sensitivity information, which is information regarding sensitivity of the subject to aberration, on the basis of the acquired line-of-sight information and the aberration distribution.

2. The terminal device according to claim 1, further comprising:
a head position and posture information acquisition unit configured to acquire head position and posture information obtained by detecting movement of a head of the subject presented with at least one of the image for the left eye and the image for the right eye,
wherein the aberration sensitivity information generation unit is configured to generate the aberration sensitivity information further on the basis of the acquired head position and posture information.

3. The terminal device according to claim 1,
wherein the first aberration distribution is an aberration distribution with a line-of-sight center position of the left eye of the subject as an origin position,
the second aberration distribution is an aberration distribution with a line-of-sight center position of the right eye of the subject as an origin position, and
the terminal device further comprises:
a line-of-sight center position calculation unit configured to calculate the line-of-sight center position on the basis of the line-of-sight information of the subject.

4. The terminal device according to claim 3,
wherein the line-of-sight center position calculation unit is configured to calculate the line-of-sight center position on the basis of a frequency distribution of a line of sight indicated by the line-of-sight information of the subject.

5. The terminal device according to claim 1,
wherein the first image output unit and the second image output unit are configured to sequentially output a plurality of pieces of image information having different distribution forms of the aberration, and
the aberration sensitivity information generation unit is configured to generate the aberration sensitivity information for each distribution form.

6. The terminal device according to claim 5,
wherein the distribution forms of the aberration include a first distribution form in which a degree of aberration is different between the right and left eyes.

7. The terminal device according to claim 5,
wherein the distribution forms of the aberration include a second distribution form in which a degree of aberration is uniform within a display range.

8. The terminal device according to claim 5,
wherein the distribution forms of the aberration include a third distribution form in which a degree of aberration at a center position of the line of sight of the subject is set to be smaller than a degree of aberration in an edge portion of a display range.

9. An aberration sensitivity information generation method in which
a computer executes:
outputting image information of an image for a left eye, in which a first aberration distribution as an aberration distribution for a left eye is settable, to a left eye display unit configured to present an image to a left eye of a subject;
outputting image information of an image for a right eye, in which a second aberration distribution as an aberration distribution for a right eye is settable, to a right eye display unit configured to present an image to a right eye of the subject;
acquiring line-of-sight information obtained by detecting movement of a line of sight of the subject presented with at least one of the image for the left eye and the image for the right eye; and
generating aberration sensitivity information, which is information regarding sensitivity of the subject to aberration, on the basis of the acquired line-of-sight information and the aberration distribution.

10. A program causing
a computer to execute:
outputting image information of an image for a left eye, in which a first aberration distribution as an aberration distribution for a left eye is settable, to a left eye display unit configured to present an image to a left eye of a subject;
outputting image information of an image for a right eye, in which a second aberration distribution as an aberration distribution for a right eye is settable, to a right eye display unit configured to present an image to a right eye of the subject;
acquiring line-of-sight information obtained by detecting movement of a line of sight of the subject presented with at least one of the image for the left eye and the image for the right eye; and
generating aberration sensitivity information, which is information regarding sensitivity of the subject to aberration, on the basis of the acquired line-of-sight information and the aberration distribution.
